(19) Europäisches Patentamt
European Patent Office
Office européen des brevets

(11) EP 2 102 345 B1

(12) **EUROPÄISCHE PATENTSCHRIFT**

(45) Veröffentlichungstag und Bekanntmachung des Hinweises auf die Patenterteilung:
**16.03.2011 Patentblatt 2011/11**

(21) Anmeldenummer: **07856822.7**

(22) Anmeldetag: **18.12.2007**

(51) Int Cl.:
***C12N 15/45*** (2006.01) ***C07K 14/135*** (2006.01)
***C12N 15/85*** (2006.01) ***C12N 15/861*** (2006.01)
***A61K 39/155*** (2006.01)

(86) Internationale Anmeldenummer:
**PCT/EP2007/011090**

(87) Internationale Veröffentlichungsnummer:
**WO 2008/077527 (03.07.2008 Gazette 2008/27)**

(54) **RSV F-PROTEIN UND SEINE VERWENDUNG**
RSV F-PROTEIN AND USE THEREOF
PROTEINE F DU VIRUS RESPIRATOIRE SYNCYTIAL (RSV), ET SON UTILISATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **21.12.2006 DE 102006060799**

(43) Veröffentlichungstag der Anmeldung:
**23.09.2009 Patentblatt 2009/39**

(73) Patentinhaber:
- **Ruhr-Universität Bochum**
**44801 Bochum (DE)**
- **Pevion Biotech Ltd.**
**3063 Ittigen / Bern (CH)**

(72) Erfinder:
- **GRUNWALD, Thomas**
**44791 Bochum (DE)**
- **ÜBERLA, Klaus**
**45549 Sprockhövel (DE)**

(74) Vertreter: **Grünecker, Kinkeldey, Stockmair & Schwanhäusser Anwaltssozietät**
**Leopoldstrasse 4**
**80802 München (DE)**

(56) Entgegenhaltungen:
**WO-A-00/18929**

- **BRANIGAN PATRICK J ET AL: "Use of a novel cell-based fusion reporter assay to explore the host range of human respiratory syncytial virus F protein" VIROLOGY JOURNAL, BIOMED CENTRAL, LONDON, GB, Bd. 2, Nr. 1, 13. Juli 2005 (2005-07-13), Seite 54, XP021010899 ISSN: 1743-422X**
- **PARK E.-K. ET AL.: "Immune induction and modulation in mice following immunization with DNA encoding F protein of respiratory syncytial virus" MOLECULS & CELLS, Bd. 12, Nr. 1, 2001, Seiten 50-56, XP002476630**
- **LI XIAOMAO ET AL: "Protection against respiratory syncytial virus infection by DNA immunization" JOURNAL OF EXPERIMENTAL MEDICINE, TOKYO, JP, Bd. 188, Nr. 4, 17. August 1998 (1998-08-17), Seiten 681-688, XP002133951 ISSN: 0022-1007**
- **TREE J A ET AL: "An assessment of different DNA delivery systems for protection against respiratory syncytial virus infection in the murine model: gene-gun delivery induces IgG in the lung" VACCINE, BUTTERWORTH SCIENTIFIC. GUILDFORD, GB, Bd. 22, Nr. 19, 23. Juni 2004 (2004-06-23), Seiten 2438-2443, XP004515462 ISSN: 0264-410X**
- **TERNETTE ET AL: "Immunogenicity and efficacy of codon optimized DNA vaccines encoding the F-protein of respiratory syncytial virus" VACCINE, BUTTERWORTH SCIENTIFIC. GUILDFORD, GB, Bd. 25, Nr. 41, 27. September 2007 (2007-09-27), Seiten 7271-7279, XP022276681 ISSN: 0264-410X**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 102 345 B1

• GRÜNWALD T. ET AL.: "RSV-vaccine: Vaccine based on adenoviral vectors preventing infection with the respiratory syncytial virus". BIOVARIA 2008. http://www.biovaria.net/downloads/ BioVaria _U5.pdf. XP002476631

**Sequenzliste**

<110> Ruhr-Universität Bochum

<120> RSV-F Protein und seine Verwendung

<130> P37178

<160> 5

<170> PatentIn version 3.3

<210> 1
<211> 1725
<212> DNA
<213> respiratory syncytial virus

<400> 1

```
atggagttgc caatcctcaa agcaaatgca attaccacaa tcctcgctgc agtcacattt     60
tgctttgctt ctagtcaaaa catcactgaa gaattttatc aatcaacatg cagtgcagtt    120
agcaaaggct atcttagtgc tctaagaact ggttggtata ctagtgttat aactatagaa    180
ttaagtaata tcaaggaaaa taagtgtaat ggaacagatg ctaaggtaaa attgatgaaa    240
caagaattag ataaatataa aaatgctgta acagaattgc agttgctcat gcaaagcaca    300
ccagcagcaa acaatcgagc cagaagagaa ctaccaaggt ttatgaatta tacactcaac    360
aataccaaaa aaaccaatgt aacattaagc aagaaaagga aaagaagatt tcttggtttt    420
ttgttaggtg ttggatctgc aatcgccagt ggcattgctg tatctaaggt cctgcactta    480
gaaggagaag tgaacaagat caaaagtgct ctactatcca caaacaaggc cgtagtcagc    540
ttatcaaatg gagttagtgt cttaaccagc aaagtgttag acctcaaaaa ctatatagat    600
aaacaattgt tacctattgt gaataagcaa agctgcagaa tatcaaatat agaaactgtg    660
atagagttcc aacaaaagaa caacagacta ctagagatta ccagggaatt tagtgttaat    720
gtaggtgtaa ctacacctgt aagcacttac atgttaacta atagtgaatt attgtcatta    780
atcaatgata tgcctataac aaatgatcag aaaaagttaa tgtccaacaa tgttcaaata    840
gttagacagc aaagttactc tatcatgtcc ataataaaag aggaagtctt agcatatgta    900
gtacaattac cactatatgg tgtgatagat acaccttgtt ggaaattaca cacatcccct    960
ctatgtacaa ccaacacaaa agaagggtca aacatctgtt taacaagaac tgacagagga   1020
tggtactgtg acaatgcagg atcagtatct ttcttcccac aagctgaaac atgtaaagtt   1080
caatcgaatc gagtattttg tgacacaatg aacagtttaa cattaccaag tgaagtaaat   1140
ctctgcaatg ttgacatatt caatcccaaa tatgattgta aaattatgac ttcaaaaaca   1200

gatgtaagca gctccgttat cacatctcta ggagccattg tgtcatgcta tggcaaaact   1260
aaatgtacag catccaataa aaatcgtgga atcataaaga cattttctaa cgggtgtgat   1320
tatgtatcaa ataaaggggt ggacactgtg tctgtaggta acacattata ttatgtaaat   1380
aagcaagaag gcaaaagtct ctatgtaaaa ggtgaaccaa taataaattt ctatgaccca   1440
ttagtattcc cctctgatga atttgatgca tcaatatctc aagtcaatga gaagattaac   1500
cagagtttag catttattcg taaatccgat gaattattac ataatgtaaa tgctggtaaa   1560
tcaaccacaa atatcatgat aactactata attatagtga ttatagtaat attgttatca   1620
ttaattgctg ttggactgct cctatactgt aaggccagaa gcacaccagt cacactaagc   1680
aaggatcaac tgagtggtat aaataatatt gcatttagta actaa                   1725
```

<210> 2
<211> 1728

<212> DNA
<213> Artificial

<220>
<223> Für die Expression in humanen wirtszellen codon-optimierte, das F Protein von RSV kodierende Nukleotidsequenz

<400> 2

```
atggagctgc ctatcctgaa ggccaacgcc atcaccacaa ttctggccgc cgtgaccttc      60
tgttttgcca gcagccagaa catcaccgag gagttctacc agagcacctg tagcgccgtg     120
agcaagggct atctgagcgc cctgagaacc ggctggtaca ccagcgtgat caccatcgag     180
ctgagcaaca tcaaggagaa caagtgcaac ggcaccgacg ccaaggtgaa gctgatgaag     240
caggagctgg acaagtacaa gaacgccgtg accgaactgc agctgctgat gcagtctacc     300
cctgccgcca acaacagagc cagacgggag ctgccccggt tcatgaacta caccctgaac     360
aacaccaaga aaaccaacgt gaccctgagc aagaagcgga agcggagatt cctgggcttt     420
ctgctgggag tgggctctgc catcgcctct ggcatcgccg tgtctaaggt gctgcacctg     480
gagggagagg tgaacaagat caagagcgcc ctgctgagca ccaataaggc cgtggtgagc     540
ctgagcaatg gcgtgagcgt gctgacaagc aaggtgctgg acctcaagaa ctacatcgac     600
aagcagctgc tgcccatcgt gaacaagcag agctgccgga tcagcaacat cgagaccgtg     660
atcgagttcc agcagaagaa caaccggctg ctggagatca ccagggagtt cagcgtgaat     720
gtgggcgtga ccacccctgt gagcacctac atgctgacca acagcgagct gctgagcctg     780
atcaacgaca tgcccatcac caacgaccag aagaagctga tgtccaacaa cgtgcagatc     840
gtgcggcagc agagctacag catcatgtcc atcatcaagg aggaggtgct ggcttacgtg     900
gtgcagctgc ctctgtacgg cgtgatcgac accccttgct ggaagctgca caccagcccct     960
```

```
ctgtgcacca ccaataccaa ggagggcagc aacatctgcc tgaccaggac cgatagaggc   1020
tggtactgcg acaatgccgg cagcgtgagc ttctttccac aggccgagac ctgtaaggtg   1080
cagagcaacc gggtgttctg cgacaccatg aacagcctga ccctgccttc tgaggtgaac   1140
ctgtgcaacg tggacatctt caaccccaag tacgactgca agatcatgac cagcaagacc   1200
gacgtgagca gcagcgtgat tacaagcctg ggcgccatcg tgagctgtta cggcaagacc   1260
aagtgcaccg ccagcaacaa gaaccgcggc atcatcaaga ccttcagcaa cggctgcgac   1320
tacgtgagca acaagggcgt ggatacagtg agcgtgggca acaccctgta ctacgtcaac   1380
aagcaggagg gcaagagcct gtacgtgaag ggcgagccca tcatcaactt ctacgacccc   1440
ctggtgttcc ctagcgacga gttcgatgcc agcatcagcc aggtgaacga gaagatcaac   1500
cagagcctgg ccttcatcag gaagagcgac gagctgctgc acaatgtgaa cgccggcaag   1560
agcaccacca acatcatgat caccaccatc atcatcgtga tcatcgtcat cctgctgtcc   1620
ctgattgctg tgggcctgct gctgtactgt aaggccagaa gcacccccgt gaccctgtct   1680
aaggatcagc tgagcggcat caacaacatc gccttctcca actgataa                1728
```

<210> 3
<211> 574
<212> PRT
<213> respiratory syncytial virus

<400> 3

```
Met Glu Leu Pro Ile Leu Lys Ala Asn Ala Ile Thr Thr Ile Leu Ala
1               5                   10                  15

Ala Val Thr Phe Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Met Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Ala Asn Asn Arg Ala Arg Arg Glu Leu Pro
```

```
        100                 105                 110

Arg Phe Met Asn Tyr Thr Leu Asn Asn Thr Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Ile Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asp Lys Gln Leu Leu Pro Ile Val Asn
        195                 200                 205

Lys Gln Ser Cys Arg Ile Ser Asn Ile Glu Thr Val Ile Glu Phe Gln
    210                 215                 220

Gln Lys Asn Asn Arg Leu Leu Glu Ile Thr Arg Glu Phe Ser Val Asn
225                 230                 235                 240

Val Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
```

340 345 350

Pro Gln Ala Glu Thr Cys Lys Val Gln Ser Asn Arg Val Phe Cys Asp
355 360 365

Thr Met Asn Ser Leu Thr Leu Pro Ser Glu Val Asn Leu Cys Asn Val
370 375 380

Asp Ile Phe Asn Pro Lys Tyr Asp Cys Lys Ile Met Thr Ser Lys Thr
385 390 395 400

Asp Val Ser Ser Ser Val Ile Thr Ser Leu Gly Ala Ile Val Ser Cys
405 410 415

Tyr Gly Lys Thr Lys Cys Thr Ala Ser Asn Lys Asn Arg Gly Ile Ile
420 425 430

Lys Thr Phe Ser Asn Gly Cys Asp Tyr Val Ser Asn Lys Gly Val Asp
435 440 445

Thr Val Ser Val Gly Asn Thr Leu Tyr Tyr Val Asn Lys Gln Glu Gly
450 455 460

Lys Ser Leu Tyr Val Lys Gly Glu Pro Ile Ile Asn Phe Tyr Asp Pro
465 470 475 480

Leu Val Phe Pro Ser Asp Glu Phe Asp Ala Ser Ile Ser Gln Val Asn
485 490 495

Glu Lys Ile Asn Gln Ser Leu Ala Phe Ile Arg Lys Ser Asp Glu Leu
500 505 510

Leu His Asn Val Asn Ala Gly Lys Ser Thr Thr Asn Ile Met Ile Thr
515 520 525

Thr Ile Ile Ile Val Ile Ile Val Ile Leu Leu Ser Leu Ile Ala Val
530 535 540

Gly Leu Leu Leu Tyr Cys Lys Ala Arg Ser Thr Pro Val Thr Leu Ser
545 550 555 560

Lys Asp Gln Leu Ser Gly Ile Asn Asn Ile Ala Phe Ser Asn
565 570

<210> 4
<211> 37
<212> DNA

<213> Artificial

<220>
<223> Oligonucleotide primer (sense)

<400> 4
gatccaagct tccaccatgg agttgccaat cctcaaa 37

<210> 5
<211> 41
<212> DNA
<213> Artificial

<220>
<223> Oligonucleotide primer (antisense)

<400> 5
tcgacctcga gttagttact aaatgcaata ttatttatac c 41

## Beschreibung

### Gebiet der Erfindung

Die vorliegende Erfindung richtet sich auf ein Nukleinsäuremolekül, das für das F-Protein des Respiratorischen Syncytialvirus oder ein Fragment davon kodiert, für die Expression in einer menschlichen Zellumgebung codon-optimierte Varianten dieses Nukleinsäuremoleküls und deren Verwendung mit viralen Vektoren oder Plasmid-Vektoren als Vakzine.

### Hintergrund der Erfindung

Bereits 1957 wurde bei Kindern mit schweren Erkrankungen der unteren Luftwege ein Virus nachgewiesen, das als Respiratorisches Syncytialvirus (RSV) bezeichnet wurde. Dieser Name weist auf die Eigenschaft des Virus hin, Erkrankungen des Respirationstrakts zu erzeugen und in vitro Syncytienbildung zu induzieren.

RSV gehört zur Familie der Paramyxoviren und darin zur Unterfamilie der Pneumovirinae. RSV hat wie die anderen Vertreter dieser Familie ein nicht-segmentiertes, durchgehendes RNA-Genom in Negativstrangorientierung. Das Genom des RSV ist 15222 Basen lang und liegt im Komplex mit Proteinen als Nucleocapsid vor.

Das Virusgenom kodiert eine Reihe von Virusproteinen. Darunter sind die Membranproteine, die als RSV G-Protein und RSV F-Protein bezeichnet werden. Das G-Protein ist für die spezifische Adsorption des Viruspartikels an die Zelloberfläche verantwortlich, während das F-Protein die Fusion der viralen mit der zellulären Membran induziert. Das F-Protein wird als Vorläuferpolypeptid $F_0$ synthetisiert und besitzt am N-terminalen Ende ein Signalpeptid für den Transport des Translationskomplexes zur Membran des endoplasmatischen Reticulums. Nach dem Durchschleusen der Aminosäurekette durch die Membran, bewirkt eine hydrophobe Sequenz am C-terminalen Ende die Verankerung des $F_0$ Proteins in der Membran und das Signalpeptid wird abgespalten. In der Folge wird das Protein während des Transports durch den Golgi-Apparat glykosyliert. Ebenfalls im Golgi-Apparat erfolgt eine Spaltung des $F_0$ Proteins in den aminoterminalen $F_2$ Teil und das $F_1$ Protein. Die Spaltstelle liegt zwischen einem Abschnitt basischer Aminosäuren und einer hydrophoben Domäne. Diese hydrophobe Domäne von etwa 25 Aminosäuren Länge bildet nach der Spaltung den N-Terminus des $F_1$ Proteins und vermittelt die Verschmelzung der viralen mit der zellulären Membran nach der Absorption. Das $F_2$ Protein bleibt mit dem $F_1$ Protein über eine Disulfidbrücke verbunden.

Antikörper, die gegen dieses fusionsvermittelnde Peptid des $F_1$ Proteins gerichtet sind, können die Aufnahme des Virus in die Zelle verhindern und haben daher neutralisierende Wirkung.

Die Infektion mit dem RSV ist hochansteckend, in einem Milliliter Speichel finden sich bis zu $\mathbf{10^6}$ infektiöse Viruspartikel. Es wird vorwiegend durch Tröpfchen und direkten Kontakt mit infizierten Personen übertragen. Vor allem Kinder infizieren sich während der Wintermonate.

Das RSV gilt als das infektiologische Hauptproblem des ersten Lebensjahres. Insbesondere gefährdet sind Säuglinge im Alter zwischen sechs Wochen und einem halben Jahr. Im Alter von vier Jahren besitzen 80 % der Kinder Antikörper gegen das Virus.

Reinfektionen mit leichten Brkrankungsformen treten aber auch in höherem Alter als Folge einer absinkenden Antikörperkonzentration auf. Insbesondere häufig sind Nosokomialinfektionen in Altersheimen, Kindergärten und - kliniken.

Die Inkubationszeit des RSV betrage etwa 4-5 Tage. Die Erkrankung zeigt sich durch leichte bis schwere lebensbedrohende grippale Infekte mit Fieber und Schnupfen. Häufig kommt es auch zu Entzündungen des Rachens (Pharyngitis) und der Luftröhre (Tracheitis) sowie der Bronchien (Bronchitis).

Nach der Tröpfcheninfektion des oberen Respirationstrakts vermehrt sich das Virus in den Zellen der Schleimhaut und kann sich von dort aus innerhalb von ein bis zwei Tagen in die unteren Luftwege ausbreiten.

Bin Impfstoff gegen RSV ist zurzeit noch nicht bekannt. Durch Formalin abgetötete Viren waren wenig erfolgreich, da das p-Protein durch die chemische Behandlung zerstört wird und nur Antikörper gegen das G-Protein gebildet werden. Diese sind zwar virusneutralisierend, können aber die Ausbreitung des Virus durch Zellfusionen nicht verhindern. Eine passive Immunisierung durch Immunglobulingabe wird angewandt, ist jedoch mit hohen Kosten verbunden und eignet sich daher nicht zur prophylaktischen Impfung größerer Bevölkerungsgruppen. WO0018929 stellt Impfstoffe vor, welche ein rekombinantes F-Protein umfassen. Andere Dokumente schlagen Impfstoffe vor, die das RSV F-Protein kodierende Nukleinsäuren umfassen: Park (2001). Mol. & Cells 12(1):50-56; Li (1998), J. Exp. Med. 188(4):681-688; Tree (2004), Vaccine 22(19):2438-2443.

Es besteht daher weiterhin ein Bedarf für einen wirksamen Impfstoff gegen eine Infektion mit dem RSV.

### Zusammenfassung der Erfindung

Die vorliegende Erfindung richtet sich auf ein Nukleinsäuremolekül, das eine Nukleotidsequenz umfasst, die für das F-Protein des Respiratorischen Syncytialvirus (RSV) kodiert, und die für eine effiziente Expression des F-Proteins des RSV in einer Wirtszelle codon-optimiert wurde. Die Nukleotidsequenz, die für ein F-Protein des RSV kodiert, umfasst eine für die Expression des F-Proteins in einer menschlichen wirtszelle codon-optimierte Nukleotidsequenz von SEQ ID NO: 2.

Ausführungsform besteht die Nukleotidsequenz, die für das F Protein des RSV kodiert, aus der codon-optimierten Nukleotidsequenz SEQ ID NO: 2.

Weiterhin umfasst die vorliegende Erfindung auch einen Vektor, der eins der erfindungsgemäßen Nukleinsäuremoleküle beinhaltet. Die erfindungsgemäßen Nukleinsäuremoleküle können in dem Vektor in Form einer (Expressions)-Kassette enthalten sein, die neben der erfindungsgemäßen Nukleotidsequenz auch eine mit der Nukleotidsequenz funktional verknüpfte Transkriptions- und/oder Translationskontrollsequenz, wie beispielsweise einen Promotor, umfassen kann. Noch ein weiterer Aspekt dieser Erfindung sind Zellen, die einen solchen Vektor enthalten.

In einer bevorzugten Ausführungsform der Erfindung ist der verwendete Vektor ein viraler Vektor oder ein Plasmid. Besonders bevorzugt ist ein adenoviraler Vektor, in welchem die E1-Region zumindest teilweise deletiert wurde, so dass der adenovirale Vektor replikationsdefizient ist. Zusätzlich kann gegebenenfalls auch die E3-Region deletiert sein.

In einer Ausführungsform der Erfindung enthält der adenovirale Vektor die erfindungsgemäße Nukleotidsequenz in Form einer Expressionskassette, in der die das F Protein des RSV kodierende Nukleotidsequenz funktional mit einem geeigneten Promotor, beispielsweise dem CMV Promotor, verknüpft ist. Der verwendete Promotor im adenoviralen Vektor ist vorzugsweise regulierbar, beispielsweise ein Tetrazyklin-regulierbarer Promotor, um in der adenoviralen Produktionszelle die Expression des F-Proteins von RSV zu verhindern. In allen Zellen, die das Regulationssystem nicht enthalten, ist der Promotor aktiv und exprimiert das F-Protein von RSV. Ein solcher adenoviraler Vektor kann als Vakzin-Vektor eingesetzt werden

In einem weiteren Aspekt, umfasst die vorliegende Erfindung auch Plasmidvektoren, die die erfindungsgemäßen Nukleotidsequenzen umfassen. In einer Ausführungsform können diese Plasmidvektoren als Shuttle Vektoren eingesetzt werden und umfassen daher einen Nukleinsäureanteil der die homologe Rekombination mit einem geeigneten Rückgratplasmid, das beispielsweise ein Großteil eines viralen Genoms enthält, ermöglicht. Besonders bevorzugt sind im Shuttle Vektor adenovirale Sequenzen enthalten, die die homologe Rekombination mit einem anderen Plasmid, das den Großteil des adenoviralen Genoms enthält, erlauben. Auf diesem Weg lassen sich beispielsweise die oben genannten adenoviralen vakzin-Vektoren herstellen.

weiterhin bezieht sich die vorliegende Erfindung in einem weiteren Aspekt auf immunogene Zusammensetzungen, die das erfindungsgemäße Nukleinsäuremolekül mit SEQ ID NO: 2 umfassen. In einer solchen Zusammensetzung kann die erfindungsgemäße Nukleotidsequenz auch in Form der oben erwähnten Vektoren, vorzugsweise in Form eines adenoviralen Vakzin-Vektors, vorliegen. Ein solcher Vektor erlaubt den Transport der erfindungsgemäßen Nukleinsäuremoleküle in einen menschlichen Wirt und die Expression des kodierten Proteins. Das Protein wird dabei in einer Menge exprimiert, die ausreicht, die beabsichtigte Immunantwort auszulösen.

Die immunogenen Zusammensetzungen können abhängig von der gewünschten Art der Anwendung auch einen pharmazeutisch annehmbaren Träger und gegebenenfalls weitere Hilfsstoffe enthalten.

Ein weiterer Aspekt ist die Verwendung der erfindungsgemäßen Nukleinsäuremoleküle oder Vektoren für die Herstellung einer Impfstoffzusammensetzung für die Impfung eines Subjekts gegen durch die Infektion mit RSV hervorgerufene Erkrankungen. Dieses Subjekt ist vorzugsweise ein Mensch.

Die vorliegende Erfindung bezieht sich außerdem auf ein Verfahren zur Herstellung des RSV F-Proteins, wobei das Verfahren die Expression eines erfindungsgemäßen Nukleinsäuremoleküls in einer geeigneten Wirtszelle umfasst. Das erfindungsgemäße Nukleinsäuremolekül ist für die Expression in der Wirtszelle codon-optimiert.

**Kurze Beschreibung der Zeichnungen**

**Figur 1** zeigt die Ergebnisse einer RSV-qRT-PCR von RNA-Isolaten aus einer Broncho-alveolären Lavage (BAL) von Mäusen die mit unterschiedlichen Plasmid-DNA-Konstrukten immunisiert wurden. Die Zahlen über den Klammern geben die statistische Signifikanz des Unterschieds zwischen den Einzelergebnissen der verglichenen Gruppen an (Tukey-Test).

**Figur 2** zeigt die Ergebnisse einer RSV-qRT-PCR von RNA-Isolaten aus einer Broncho-alveolären Lavage (BAL) von Mäusen die mit unterschiedlichen Konstrukten immunisiert wurden nach Infektion mit RSV. Die Kopienzahl von RSV nach qRT-PCR wurde durch RNA-Quantifizierung standardisiert und ist hier für die RNA-Isolate der gewonnenen BALs dargestellt. Gezeigt sind jeweils die Einzelwerte von 6 Mäusen (schwarze Symbole) sowie der Mittelwert (Balken).

**Figur 3** zeigt die Ergebnisse eines IgG-Antikörper-ELISAs. Die vor der ersten Immunisierung (prä), nach der zweiten Immunisierung (post) und nach dem Challenge (ca. 1 x $10^7$ iE (infektiöse Einheiten) von Plaque aufgereinigtem RSV intranasal) am Tötungstag (nC) gewonnenen Serumproben der mit unterschiedlichen Zusammensetzungen (AdV-$F_{syn}$: für das synthetische (codon-optimierte) F-Protein von RSV kodierende Nukleinsäure in adenoviralem Vektor; AdV-Ova: adenoviraler Vektor mit Nukleinsäure, die für Ovalbumin kodiert; pcDF$_{syn\ ED}$: Plasmid, das für die Ektodomäne des synthetischen (cödonoptimierten) F-Proteins von RSV kodiert) immunisierten bzw. nicht immunisierten Mäuse wurden im IgG-Antikörper-ELISA auf RSV-spezifische IgG1- und IgG2a-Antikörper getestet. Die Stärke der Absorption bei der Wellenlänge 405 entspricht dem Antikörpertiter im Blut der Mäuse. Gezeigt sind jeweils die Einzelwerte von 6 Mäusen (schwarze Symbole) sowie der Mittelwert (Balken).

**Figur 4** zeigt das Ergebnis eines Neutralisationstests. Die vor der ersten Immunisierung (prä), nach der zweiten Immunisierung (post) und nach der Challenge am Tötungstag (nC) gewonnenen Serumproben der Mäuse wurden seriell verdünnt und dann die Verdünnungsreihe im Neutralisationstest auf neutralisierende Antikörper gegen RSV getestet. Dargestellt ist jeweils die höchste Serumverdünnung, bei der die Infektion durch RSV durch neutralisierende Antikörper zu 50% (IC50) gehemmt wird. Gezeigt sind jeweils die Einzelwerte von 6 Mäusen (schwarze Symbole) sowie der Mittelwert (Balken).

**Figur 5** zeigt eine RSV-qRT-PCR von RNA-Isolaten aus einer Brocho-alveolären Lavage (BAL) von Mäusen, die über unterschiedliche Verabreichungswege mit einem erfindungsgemäßen adenoviralen Vektor, der ein für ein codon-optimiertes RSV-F Protein kodierendes Nukleinsäuremolekül umfasst, immunisiert wurden. Die Kopienzahl von RSV nach qRT-PCR wurde durch RNA-Quantifizierung standardisiert und ist hier für die RNA-Isolate der am Tötungstag gewonnenen BALs dargestellt. Gezeigt sind jeweils die Einzelwerte von 6 Mäusen (schwarze Symbole) sowie der Mittelwert (Balken).

**Figur 6** zeigt eine RSV-qRT-PCR von RNA-Isolaten aus dem Lungenhomogenat von Mäusen, die über unterschiedliche Verabreichungswege mit einem erfindungsgemäßen adenoviralen Vektor, der ein für ein codon-optimiertes RSV-F Protein kodierendes Nukleinsäuremolekül umfasst, immunisiert wurden. Es sind die Ergebnisse der qRT-PCR von den RNA-Isolaten der am Tötungstag gewonnenen Lungenhomogenate dargestellt. Die Kopienzahl von RSV nach qRT-PCR wurde durch RNA-Quantifizierung standardisiert und in Bezug zum RNA-Gehalt der Isolate umgerechnet. Gezeigt sind jeweils die Einzelwerte von 5 (*) oder 6 Mäusen (schwarze Symbole) sowie der Mittelwert (Balken).

**Figur 7** zeigt einen IgG-Antikörper-ELISA von Mäusen, die über unterschiedliche Verabreichungswege mit einem erfindungsgemäßen adenoviralen Vektor, der ein für ein codon-optimiertes RSV-F Protein kodierendes Nukleinsäuremolekül umfasst, immunisiert wurden. Die vor der ersten Immunisierung (prä), nach der ersten Immunisierung (post I), nach der zweiten Immunisierung (post II) und nach dem Challenge am Tötungstag (nC) gewonnenen Serumproben der Mäuse wurden im IgG-Antikörper-ELISA auf RSV-spezifische IgG1- und IgG2a-Antikörper getestet. Die Stärke der Absorption bei der Wellenlänge 405 entspricht dem Antikörpertiter im Blut der Mäuse. Gezeigt sind jeweils die Einzelwerte von 5 (*) oder 6 Mäusen (schwarze Symbole) sowie der Mittelwert (Balken).

**Figur 8** zeigt das Ergebnis eines Neutralisationstests von Mäusen, die über unterschiedliche Verabreichungswege mit einem erfindungsgemäßen adenoviralen Vektor, der ein für ein codon-optimiertes RSV-F Protein kodierendes Nukleinsäuremolekül umfasst, immunisiert wurden. Die vor der ersten Immunisierung (prä), nach der ersten Immunisierung (post I), nach der zweiten Immunisierung (post II) und nach dem Challenge am Tötungstag (nC) gewonnenen Serumproben der Mäuse wurden seriell verdünnt und dann die Verdünnungsreihe im Neutralisationstest auf neutralisierende Antikörper gegen RSV getestet. Dargestellt ist jeweils die höchste Serumverdünnung, bei der die Infektion durch RSV durch neutralisierende Antikörper zu 50% (IC50) gehemmt wird. Gezeigt sind jeweils die Einzelwerte von 5 (*) oder 6 Mäusen (schwarze Symbole) sowie der Mittelwert (Balken).

**Figur 9** zeigt die Ergebnisse einer RSV-qRT-PCR von RNA-Isolaten aus der Broncho-alveolären Lavage (BAL) von Mäusen nach subkutaner Immunisierung mit einem erfindungsgemäßen adenoviralen Vektor, der ein für ein codon-optimiertes RSV-F Protein kodierendes Nukleinsäuremolekül umfasst (AdV-$F_{syn}$), in verschiedenen Dosierungen (Dosiseskalation). Die Kopienzahl von RSV nach qRT-PCR wurde durch RNA-Quantifizierung standardisiert und ist für die RNA-Isolate der am Tötungstag gewonnenen BALs dargestellt. Gezeigt sind jeweils die Einzelwerte von 5 (*) oder 6 Mäusen (schwarze Symbole) sowie der Mittelwert (Balken).

**Figur 10** zeigt die Ergebnisse einer RSV-qRT-PCR von RNA-Isolaten aus dem Lungenhomogenat von Mäusen nach subkutaner Immunisierung mit einem erfindungsgemäßen adenoviralen Vektor, der ein für ein codon-optimiertes RSV-F Protein kodierendes Nukleinsäuremolekül umfasst (AdV-$F_{syn}$), in verschiedenen Dosierungen (Dosiseskalation). Es sind die Ergebnisse der qRT-PCR von den RNA-Isolaten der am Tötungstag gewonnenen Lungenhomogenate dargestellt. Die RSV-Kopienzahl nach qRT-PCR wurde durch RNA-Quantifizierung standardisiert und in Bezug zum RNA-Gehalt der Isolate umgerechnet. Gezeigt sind jeweils die Einzelwerte von 5 (*) oder 6 Mäusen (schwarze Symbole) sowie der Mittelwert (Balken).

**Figur 11** zeigt das Ergebnis eines IgG-Antikörper-ELISAs von Mäusen nach subkutaner Immunisierung mit einem erfindungsgemäßen adenoviralen Vektor, der ein für ein codon-optimiertes RSV-F Protein kodierendes Nukleinsäuremolekül umfasst (AdV-$F_{syn}$), in verschiedenen Dosierungen (Dosiseskalation). Die vor der ersten Immunisierung (prä), nach der ersten Immunisierung (post I), nach der zweiten Immunisierung (post II) und nach dem Challenge am Tötungstag (nC) gewonnenen Serumproben der Mäuse wurden im IgG-Antikörper-ELISA auf RSV-spezifische IgG1- und IgG2a-Antikörper getestet. Die Stärke der Absorption bei der Wellenlänge 405 entspricht dem Antikörpertiter im Blut der Mäuse. Gezeigt sind jeweils die Einzelwerte von 5 (*) oder 6 Mäusen (schwarze Symbole) sowie der Mittelwert (Balken).

**Figur 12** zeigt das Ergebnis eines Neutralisationstests von Mäusen nach subkutaner Immunisierung mit einem erfindungsgemäßen adenoviralen Vektor, der ein für ein codon-optimiertes RSV-F Protein kodierendes Nukleinsäuremolekül umfasst (AdV-$F_{syn}$), in verschiedenen Dosierungen (Dosiseskalation). Die vor der ersten Immunisierung (prä), nach der ersten Immunisierung (post I), nach der zweiten Immunisierung (post II) und nach dem Challenge am Tötungstag (nC) gewonnenen Serumproben der Mäuse wurden seriell verdünnt und dann die Verdünnungsreihe im Neutralisationstest auf neutralisierende Antikörper gegen RSV getestet. Dargestellt ist jeweils die höchste Serumverdünnung, bei der die Infektion durch RSV durch neutralisierende Antikörper zu 50% (IC50) gehemmt wird. Gezeigt sind jeweils die Einzelwerte von 5 (*) oder 6 Mäusen (schwarze Symbole) sowie der Mittelwert (Balken).

**Figur 13** zeigt die Vektorkarte eines Shuttleplasmids, in das über die Restriktionsstellen HindIII und XhoI das codonoptimierte RSV-F einkloniert wurde (pS-DP-Fsyn), bzw. eines Shuttleplasmids, das zur Aufnahme von codon-optimierten RSV-F verwendet wurde, und das einen Tetrazyklin-abhängigen Promotor enthält (pS-DP-delta). Dieser Promotor wird über ein genetisches Schalterelement in 293TRex-Zellen (Invitrogen) ausgeschaltet.

**Figur 14** zeigt Expressionsunterschiede von pFwt (Expressionsplasmid mit der Wildtyp Sequenz des RSV-F Proteins), pIFwt (Expressionsplasmid mit der Wildtyp Sequenz des RSV-F Proteins mit einem zusätzlichen Intron vor dem offenen Leserahmen), pFsyn (Expressionsplasmid mit der codon-optimierten Sequenz des RSV-F Proteins) und pIFsyn (Expressionsplasmid mit der codon-optimierten Sequenz des RSV-F Proteins mit einem zusätzlichen Intron vor dem offenen Leserahmen) nach Transfektion in Hep2 Zellen, Lyse der Zellen 48 Stunden nach der Transfektion, Auftrennung der Proteine des Zelllysats mittels Gelelektrophorese, Transfer auf eine Nitrocellulosemembran und Detektion mittels eines spezifischen Antikörpers (Ab: anti-RSV-F). pcDNA3.1: Plasmid ohne Fremdgen (Leerplasmid); Hep2: Zellen ohne Plasmid; RSV: mit RSV infizierte Zellen (Positivkontrolle); kD: Molekulargewicht in Kilodalton.

Figur 15 zeigt eine Analyse der Expressionsstärken. Die Expression des codon-optimierten Konstrukts des RSV-F Proteins mit vorgeschaltetem Intron (pIFsyn) wurde durch serielle Verdünnung (1:$10^2$bis 1:$10^4$) des Zelllysates mit der Expression des Plasmids mit der Wildtyp Sequenz mit vorgeschaltetem Intron (pIFwt) im unverdünnten Zelllysat (1:1) verglichen. Die Proteinmenge von RSV-F durch Expression des codon-optimierten Konstrukts liegt bei einer Verdünnung von 1000-fach (1:$10^3$) noch deutlich über der durch das Plasmid mit der ursprünglichen Sequenz. (kDa = Molekulargewicht in Kilodalton).

## Detaillierte Beschreibung der Erfindung

Gegenstand der vorliegenden Erfindung ist in einem ersten Aspekt ein Nukleinsäuremolekül, das eine Nukleotidsequenz umfasst, die für das F Protein des Respiratorischen Syncytialvirus (RSV) kodiert, wobei diese Nukleotidsequenz SEQ ID NO: 2 umfasst. Diese Sequenz unterscheidet sich von den bekannten das F Protein von RSV kodierenden Sequenzen durch eine Substitution eines Nukleotids in der kodierenden Sequenz. Diese Substitution führt zu einer geänderten Aminosäuresequenz des F Proteins von RSV, in der sich an Position 241 ein Valin anstelle eines Alanins befindet. Dieses Nukleinsäuremolekül kann als Impfstoff oder Teil einer Impfstoffzusammensetzung verwendet werden.

"Fragment" im Zusammenhang mit einer Nukleinsäure bezieht sich auf Teile einer Nukleinsäuresequenz, die gegenüber dieser 3'- und/oder 5'-verkürzt sind. Insbesondere sind solche Fragmente mindestens 30, vorzugsweise mindestens 60, noch bevorzugter mindestens 100 Nukleotide lang und kodieren einen Teil eines RSV F Proteins, der eine ausreichende Immunogenität besitzt, um in einem Organismus eine Immunantwort auszulösen.

"Immunogen" oder "Immunogenität" bezieht sich auf die Fähigkeit einer Substanz, beispielsweise eines Peptids oder Proteins, in einem Organismus eine Immunantwort hervorzurufen. Die Immunogenität einer Substanz kann beispielsweise über den Nachweis von Antikörpern bestimmt werden.

Die Erfindung richtet sich auf synthetische Nukleinsäuremoleköle, die für die Expression in einem Wirtsorganismus, einem Wirtsgewebe oder einer Wirtszelle codon-optimiert wurden.

"Synthetisch" im Zusammenhang mit einer Nukleinsäure bedeutet im Kontext der vorliegenden Erfindung, dass es sich um ein Nukleinsäuremolekül handelt, das nicht natürlich vorkommt. Ein solches synthetisches Nukleinsäuremolekül kann beispielsweise ein codon-optimiertes Nukleinsäuremolekül sein.

"Codon-optimiert" im Zusammenhang mit einem Nukleinsäuremolekül bezieht sich auf ein Nukleinsäuremolekül, dessen Nukleotidsequenz so verändert worden ist, dass die Expression in einem wirtsorganismus verbessert wird, d.h. eine größere Menge des durch das Nukleinsäuremolekül kodierten Proteins hergestellt wird.

In der Vergangenheit waren Versuche das F Protein des Respiratorischen Syncytial Virus (RSV) durch Transfektion mit Polymerase II abhängigen DNA Expressionsplasmiden zu exprimieren nicht erfolgreich.

Eukaryotische zellen unterscheiden sich von prokaryotischen Zellen durch eine stärkere Kompartimentalisierung des Intrazellularraums, was dazu dient komplexe enzymatische Reaktion, die für eine effiziente Proteinexprension, den Zellmetabolismus und/oder die Zellteilung notwendig sind, zu ermöglichen. In die Anpassung an die Wirtszelle und insbesondere an deren Expressionsmaschinerie ist der Schlüssel für die Replikation jedes Virus. RNA Viren, die sich im Cytoplasma ihrer Wirtszellen replizieren, haben sich unter den Umgebungsbedingungen des Cytoplasmas entwickelt. Diese Viren besitzen daher ihre eigene Transkriptionsmaschinerie, die eine RNA-abhängige RNA Polymerase, die zu der Synthese von mRNA aus der genomischen RNA führt, umfasst. Aus diesem Grund sind cytoplasmatische RNA Viren nicht an das komplexe nukleäre Milieu der eukaryotischen Wirtszelle angepasst. Die ineffiziente Expression von viralen Genen unter der Kontrolle eines eukaryotischen Promotors kann daher durch das Fehlen von Elementen, die für die Stabilisierung der prä-mRNA, die mRNA-Prozessierung und/oder den Export aus dem Kern erforderlich sind, erklärt werden. Das Ändern der Codons von viralen Genen zu den Codons, die am häufigsten von der Wirtszelle verwendet werden, erlaubt die wirksame Expression von viralen Genen. Aus diesem Grund wurde die Nukleinsäuresequenz mit der SEQ ID NO: 1 in übereinstimmung mit dieser Erfindung in eine synthetische, codon-optimierte Sequenz umgewandelt, die zu einer identischen translatierten Sequenz führt, aber alternative Codons verwendet. Das Verfahren der Codon-Optimierung umfasst dabei die folgenden Schritte: Identifizierung der Platzierung der Codons für einen exakten offenen Leserahmen, Vergleich der Codons mit der verwendungshäufigkeit in dem gewünschten Wirtsorganismus, falls das Codon nicht das am häufigsten verwendete ist, Ersetzen durch das optimale Codon für die Expression, Wiederholen dieses Vorgangs für das gesamte Gensegment, Überprüfen der neuen Gensequenz auf unerwünschte Sequenzen, die durch den Codon-Austausch erzeugt wurden, wie beispielsweise Poly-Adenylierungsstellen, unerwünschte Restriktionsstellen, Intron-Splice-Erkennungsstellen etc., Zusammensetzen der synthetischen Gensegmente und Testen der Expression in einem Wirtsorganismus oder einer Wirtszelle.

Eine mittels dieses Verfahrens erhaltene, für die Expression in menschlichen Zellen codon-optimierte, synthetische Nukleinsäuresequenz ist ein Nukleinsäuremolekül, das die Nukleötidsequenz von SEQ ID NO: 2 umfasst oder aus dieser besteht.

Im Vergleich zu der Wildtypsequenz mit SEQ ID NO: 1 konnten die Erfinder bei der Verwendung der Nukleotidsequenz von SEQ ID NO: 2 in menschlichen Zellen eine um den Faktor 1000 verbesserte Expression des F Proteins von RSV beobachten.

Die codon-optimierten Sequenzen werden dann in eine Expressionskassette eingebaut, die zusätzlich Sequenzen umfasst, die die effiziente Expression des kodierten Proteins in einer Wirtszelle, vorzugsweise einer menschlichen Zelle, erlauben. Eine solche Expressionskassette enthält dann die codon-optimierte Nukleotidsequenz mit damit funktional verknüpften zugehörigen Transkriptions- und Translationskontrollsequenzen, wie beispielsweise einem Promotor und/oder Terminierungssequenzen.

Die Bezeichnung "Kassette" oder "Expressionskassette" bezieht sich auf die erfindungsgemäßen Nukleotidsequenzen, die die Nukleotidsequenzen enthalten, die exprimiert werden sollen. Wegen entsprechender Restriktionsstellen an den 5'- und 3'-Enden der Sequenzen kann diese Kassette/Expressionskassette einfach in einen Vektor oder ein Plasmid eingeschoben, entfernt oder ersetzt werden. Die Expressionskassette enthält in der Regel zusätzlich zu der kodierenden Nukleotidsequenz Transkriptions- und Translationskontrollsequenzen, wie beispielsweise einen Promotor und/oder Terminierungssequenzen, die mit der Nukleotidsequenz funktional verbunden sind und die Expression in einer Wirtszelle erlauben.

Die Bezeichnung "Promotor" in Sinne der vorliegenden Erfindung bezieht sich auf eine Region auf einem DNA-Strang, der die Erkennung durch die RNA Polymerase ermöglicht. Nach der Erkennung der Promotorregion durch eine RNA-Polymerase wird durch die Bindung der RNA-Polymerase ein Initiationskomplex, um die Transkription einzuleiten. Dieser Komplex kann durch zusätzliche aktivierende Sequenzen (Enhancer) oder hemmende Sequenzen (Silencer) zusätzlich moduliert und reguliert werden.

Ein für die Expression der erfindungsgemäßen Nukleotidsequenzen geeigneter Promotor ist beispielsweise der CMV (Cytomegalievirus) Promotor. Dieser kann in einer Expressionskassette für die Expression in menschlichen Zellen funktional mit der erfindungsgemäßen Nukleotidsequenz verknüpft sein.

Eine solche Expressionskassette, die die erfindungsgemäße Nukleotidsequenz in Verbindung mit zusätzlichen für die Expression in einer Wirtszelle erforderlichen Sequenzen enthält, kann in einen Vektor eingefügt werden.

Die Bezeichnung "Vektor" bezieht sich auf Mittel, mit denen DNA-Moleküle in einen Wirtsorganismus oder ein Wirtsgewebe oder eine Wirtszelle eingebracht werden können. Es gibt verschiedene Arten von Vektoren, die beispielsweise Plasmide, Cosmide, Viren, z.B. Adenoviren, und Bakteriophagen einschließen.

Ein so erhaltener Vektor ist ebenfalls ein Bestandteil der vorliegenden Erfindung. Der Vektor ist vorzugsweise ein Plasmid oder ein viraler Vektor, insbesondere ein adenoviraler Vektor.

Ein solcher adenoviraler Vektor, der die erfindungsgemäße Nukleotidsequenz enthält, kann eine nicht-funktionelle E1-Genregion besitzen, um die Replikation des Virus und die Erzeugung infektiöser Viruspartikel in einer Wirtszelle zu verhindern. Vorzugsweise ist die E1-Genregion deletiert und/oder durch die eingefügte Expressionskassette ersetzt. Zusätzlich kann ein solcher Vektor auch eine Deletion der E3-Genregion aufweisen, die normalerweise für das Umgehen der Wirtsimmunität verantwortlich ist. Solche rekombinanten Adenoviren sind z. B. aus US 5,922,576 bekannt und können in bekannten Zelllinien ("packaging line"), wie z.B. 293-, 911- oder PerC.6-Zellen, die das virale E1-Gen exprimieren, vervielfältigt werden.

Um einen solchen adenoviralen Vektor herzustellen, kann die Expressionskassette, die die erfindungsgemäße Nukleotidsequenz umfasst, in einen Plasmidvektor eingefügt werden, z.B. über geeignete Restriktionsschnittstellen. Ein solcher Plasmidvektor kann adenovirale Nukleinsäuresequenzen umfassen, die die homologe Rekombination mit einem geeigneten Rückgratplasmid, das einen Großteil des adenoviralen Genoms enthält, ermöglichen. Durch die Rekombination erhält man dann einen adenovirales Transfer-(Shuttle)-Plasmid, das die erfindungsgemäße Nukleotidsequenz in Form einer Expressionskassette enthält. Ein solches adenovirales Transfer-(Shuttle-)Plasmid kann beispielsweise auch eine regulierte Expression der eingesetzten erfindungsgemäßen Nukleotidsequenz vermitteln. Dadurch kann dann eine große Menge an adenoviralen Vektoren hergestellt werden. Die adenoviralen Vektoren können im Folgenden dann als vakzin-Vektor verwendet werden.

Zur Herstellung von adenoviralen Vektoren, die das F-Protein exprimieren, kann der CMV-Promotor durch einen regulierbaren Promotor ersetzt werden. Damit kann der Vektor in Zellen expandiert werden, die ein Regulationssystem für die Expression des RSV F-Proteins, beispielsweise das Tetrazyklin regulierte Expressionsystem (293TRex Zellen), enthalten. Für die Herstellung und Expansion des adenoviralen Vektors werden daher beispielsweise 293TRex Zellen verwendet.

Regulierbare Promotoren sind Promotoren, die durch Substanzen, z.B. Antibiotika oder eines Proteins, das eine bestimmte DNA-Sequenz bindet, eine veränderte Expression des nachgeschalteten Transgen vermitteln. Solche regulierbaren Promotoren sind beispielsweise ein CMV-Promotor mit Bindestellen für den Tetrazyklin-Repressor (Tetrazyklin-regulierbarer Promotor) oder über das Steroid Ecdyson vermittelte regulierbare Expression, aber auch andere regulierbare Expressionssysteme.

Daher umfasst die vorliegende Erfindung in einem Aspekt auch Plasmide, beispielsweise oben beschriebene Shuttle-Plasmide, und adenovirale Vektoren, die die erfindungsgemäßen Nukleotidsequenzen enthalten. Vorzugsweise enthält der adenovirale Vektor die Nukleotidsequenz mit SEQ ID NO: 2 in Form einer Expressionskassette mit einem Promotor, beispielsweise dem CMV Promotor. Besonders bevorzugt ist ein solcher erfindungsgemäßer Vektor der adenovirale Vektor AdV-$F_{syn}$.

Die molekularbiologischen Standardtechniken zur Herstellung und Reinigung der DNA-Konstrukte der Erfindung, zur Herstellung der Adenoviren und adenoviralen Vektoren und der (Shuttle-) Plasmide sind dem Fachmann aus dem Stand der Technik bekannt.

Die adenoviralen Vektoren oder Plasmide, die die erfindungsgemäße Nukleotidsequenz enthalten, können einem Subjekt, beispielsweise einem Menschen, verabreicht werden, um eine Immunantwort gegen RSV zu induzieren.

Aus diesem Grund bezieht sich die vorliegenden Erfindung in einem weiteren Aspekt auf immunogene Zusammensetzungen, die das erfindungsgemäße Nukleinsäuremolekül mit SEQ ID NO: 2 umfassen. In einer solchen Zusammensetzung kann die erfindungsgemäße Nukleotidsequenz in Form der oben erwähnten Plasmide oder Vektoren, vorzugsweise in Form' eines adenoviralen Vektors, vorliegen. Ein solcher Vektor erlaubt den Transport der erfindungsgemäßen Nukleinsäuremoleküle in einen menschlichen Wirt und die Expression des kodierten Proteins. Das Protein wird dabei in einer Menge exprimiert, die ausreicht die beabsichtigte Immunantwort auszulösen.

Die immunogenen Zusammensetzungen können abhängig von der gewünschten Art der Anwendung auch einen pharmazeutisch annehmbaren Träger- und/oder Hilfsstoff enthalten. Hilfsstoffe umfassen unter anderem auch bekannte Adjuvantien.

Die Verabreichung der immunogenen Zusammensetzungen kann dabei auf dem Fachmann bekanntem Weg erfolgen und schließt orale Dosierungsformen, wie z.B. Tabletten, Kapseln, Pulver, Granulate, Lösungen, Suspensionen, Sirups und Emulsionen, oder alternativ Injektionen, beispielsweise intravenös, intraperitoneal, subkutan oder intramuskulär, ein. Ebenfalls möglich sind die intranasale oder inhalative Verabreichung. Alle diese Verabreichungswege sind dem Fachmann auf dem Gebiet bekannt. Bevorzugte Verabreichungswege sind oral, intranasal, inhalativ, subkutane oder intramuskuläre Injektion

Die Formulierung der immunogenen Zusammensetzungen in einer Form, die für die gewünschte Art der Verabreichung geeignet ist, ist dem Fachmann bekannt rund kann beispielsweise Remington: The Science and Practice of Pharmacy ("Remington's Pharmaceutical Sciences") von Gennaro A.R., 20te Ausgabe, 2000: Williams & Wilkins PA, USA, entnommen werden. Beispielsweise können die erfindungsgemäßen Nukleinsäuremoleküle in einer physiologisch akzeptablen Lösung, wie z.B. steriler Köchsalzlösung oder steril gepufferter Salzlösung vorliegen.

Eingeschlossen ist daher auch die Verwendung der erfindungsgemäßen Nukleinsäuremoleküle, Plasmid oder Vektoren für die Herstellung einer Impfstoffzusammensetzung für die Impfung eines Subjekts gegen durch die Infektion mit RSV hervorgerufene Erkrankungen. Dieses Subjekt ist vorzugsweise ein Mensch. Die Impfung kann beispielsweise in einem "prime and boost"-Verfahren erfolgen.

Die Menge an exprimierbarer DNA, die bei einer Impfung in den Empfänger eingebracht werden soll, hängt teilweise von der Stärke des verwendeten Promotors und der Immunogenität des exprimierten Genprodukts ab. Im Allgemeinen wird für eine immunologisch oder prophylaktisch effektive Dosis eines Plasmid-Vakzin-Vektors eine Dosis von 1 ng bis 100 mg, vorzugsweise von ungefähr 10 $\mu$g bis 300 $\mu$g, direkt in das Muskelgewebe verabreicht. Eine effektive Dosis für rekombinante Adenoviren sind in etwa $10^6$-$10^{12}$ Partikel, bevorzugt $10^7$-$10^{11}$ Partikel.

Ein Verfahren zur Herstellung des RSV F-Proteins, wobei das Verfahren die Expression eines erfindungsgemäßen Nukleinsäuremoleküls in einer geeigneten Wirtszelle umfasst ist ebenfalls Bestandteil der vorliegenden Erfindung. Das erfindungsgemäße Nukleinsäuremolekül ist für die Expression in der wirtszelle codon-optimiert. Dieses Verfahren umfasst die Transfektion einer erfindungsgemäßen Nukleinsäuresequenz, beispielsweise in Form eines Plasmids, in eine geeignete Wirtszelle, Expression des RSV-F Proteins in der wirtszelle und, gegebenenfalls, die Isolierung und Reinigung des RSV-F Proteins aus den Zellen. Das verfahren ist vorzugsweise ein *in vitro* verfahren.

Die nachfolgenden Beispiele dienen der genaueren Veranschaulichung der Erfindung.

## Beispiele

### Beispiel 1: Kultivierung von RSV

Die Passage von RSV wurde mit Hep2 Zellen durchgeführt und bei -80°C gelagert. Hep2 oder 293T Zellen wurden durch die Zugabe von RSV-enthaltendem Zellüberstand infiziert. Zwei Stunden nach der Zugabe des Virus wurden die Überstände entfernt und die Zellen mit DMEM Medium, das 0,5% fötales Kälberserum (FCS) und 100 $\mu$g/ml Penicillin G und Streptomycinsulfat enthielt, versorgt.

### Beispiel 2: Herstellung des RSV-F Expressionsplasmids

Für die Herstellung des RSV-F Expressionsplasmids, wurde unter Verwendung des Qiagen RNeasy® Mini Kits aus RSVinfizierten Hep2 Zellen cytoplasmatische RNA isoliert. Nach der reversen Transkription (ThermoScript™ RT-PCR System, Invitrogen) wurde die RSV-F cDNA über PCR (Primers: sense: 5'- [G A T C C A A G C T T C C A C C] A T G G A G T T G C C A A T C C T C A A A; antisense : 5'- [T C G A C C T C G A G] T T A G T T A C T A A A T G C A A T A T T A T T T A T A C C) unter Verwendung der Platinum® Taq DNA-polymerase (Invitrogen) amplifiziert. Das 1.7 kb Fragment wurde in den pcDNA3.1(+) Vektor (Invitrogen) umkloniert.

Die Codon-Optimierung des offenen Leserahmens (ORF) des Wildtyps wurde von der Firma Geneart (Regensburg, Deutschland) durchgeführt. Dabei wurden synthetisch hergestellte Oligonukleotide zusammengefügt und das resultierende Fragment in das pUC57 Plasmid einkloniert und sequenziert.

Der codon-optimierte ORF wurde in den pcDNA3.1(+) Vektor (Invitrogen) und den pI Vektor über HindIII / XhoI Restriktion umkloniert. Die Deletion des Stop Codons des RSV-F ORF wurde mit PCR Mutagenese durchgeführt. Die Sequenz aller Plasmide wurde über eine Sequenzanalyse (Genterprise, Mainz, Deutschland) bestätigt.

### Beispiel 3: Zelltransfektion

293T und Hep2 Zellen wurden in Dulbecco's modified Eagle's medium (Invitrogen) ergänzt mit 10% fötalem Kälberserum (Invitrogen), Penicillin G and Streptomycinsulfat in einer Endkonzentration von jeweils 100 $\mu$g/ml kultiviert. Die Zellen wurden in 25 $cm^2$ Kolben mit 5 $\mu$g der in Beispiel 2 hergestellten Plasmid-DNA mittels der Calciumphoshat Kopräzipitationsmethode, wie von DuBridge et al. (DuBridge et al. (1987) Analysis of mutation in human cells by using an Epstein-Barr virus shuttle System. Mol. Cell Biol., 7, 379-387) beschrieben, transfiziert.

**Beispiel 4: Homologe Rekombination mittels Elektroporation und Herstellung elektrokompetenter BJ5183-Bakterien**

Zur Herstellung von rekombinanten adenoviralen Vektoren wurde die Methode der homologen Rekombination eingesetzt. Es wurden ein linearisierter Shuttle-Vektor (Figur 13) und ein adenoviraler Vektor (pAdEasy-1; Q BIOgene, Carlsbad, CA, USA) mittels Elektroporation kotransformiert. Aufgrund homologer Arme in den Plasmiden erfolgte eine Rekombination in BJ5183 Bakterien; das entstehende Plasmid trug eine Kanamycin-Resistenz, wodurch positive Klone auf einer $LB_{kan}$-Agarplatte selektioniert werden konnten. Ein solches Vektorsystem ist beispielsweise auch unter dem Namen AdEasy™ von Q BIOgene (Carlsbad, CA, USA) erhältlich.

Zur Herstellung elektrokompetenter BJ5183-Bakterien wurde eine frische Kolonie oder ein gefrorener Stock der Bakterien wurde über Nacht in 10 ml LB-Medium mit Streptomycin angezogen; diese Vorkultur wurde verwendet, um eine 1 1-Kultur anzuimpfen. Die Bakterien wurden bei 37°C im Horizontalschüttler bei 200 - 220 rpm bis zu einer $OD_{600}$ von 0,6 - 0,8 (ca. 3 Stunden) geschüttelt. Danach wurden die Zellen in Zentrifugationsbecher gesammelt und 60 min auf Eis inkubiert. Nach der Inkubation wurden die Zellen bei 2.600 g und 4°C für 10 min pelletiert, dann mit 11 eiskaltem Wasser mit 10% Glycerol gewaschen und für 30 min pelletiert. Nach Wiederholen des Waschschrittes wurden die Zellen in 20 ml Restvolumen resuspendiert, in ein 50 ml-Gefäß überführt und erneut für 10 min pelletiert. Nun wurden die Zellen in 3 - 5 ml Restvolumen resuspendiert, aliquotiert, in flüssigem Stickstoff eingefroren und bei -80°C gelagert.

Für die homologe Rekombination wurden auf Eis in einer gekühlten Elektroporations-Küvette 40 μl BJ5183-Bakterien vorgelegt und etwa 100 ng Plasmid-DNA des adenoviralen Vektors und etwa 1 μg Plasmid-DNA des Shuttle-Vektors dazu pipettiert. Die Elektroporation erfolgte bei einer Spannung von 2500 V. Nach Zusatz von 300 μl SOC-Medium wurden die Bakterien bei 37°C für 1 h in einem Schüttelapparat inkubiert und anschließend auf $LB_{kan}$-Platten über Nacht ausplattiert.

Von den gewachsenen Klonen wurde die DNA in analytischem Maßstab isoliert und überprüft. Bei positiver Rekombination wurde sie in DH5α-Bakterien retransformiert, um weitere Rekombinationsereignisse zu verhindern. Dazu wurden 50 μl DH5a Bakterien mit 1 μl DNA versetzt und elektroporiert.

**Beispiel 5: Präparative Gewinnung der in vitro klonierten oder in BJ5183-Bakterien rekombinierten Plasmid-DNA**

Zur präparativen Gewinnung der *in vitro* klonierten oder in BJ5183-Bakterien rekombinierten Plasmid-DNA dienten die Escherichia coli Stämme XL2-Blue (Stratagene) und DH5α (Invitrogen). Die verwendeten Plasmide ermöglichten mittels eines Resistenzgens die Selektion durch entsprechende Antibiotika (Ampicillin oder Kanamycin). Die transformierten Bakterien wurden auf LB-Agarplatten und in LB-Flüssigmedium mit einem Zusatz des jeweiligen Antibiotikums selektiert. Als Vorkultur für präparative Plasmidisolierungen dienten 3 ml Übernachtkulturen, die bei 37°C in einem Horizontalschüttler inkubiert wurden. Zur Extraktion größerer Mengen an DNA wurden 100 ml oder 250 ml LB-Medium in sterilen Erlenmeyerkolben mit 1 ml einer Vorkultur angeimpft und bei 37°C über Nacht im Horizontalschüttler bei 200 - 220 rpm inkubiert.

**Beispiel 6: calciumphosphat-Transfektion von Zellen**

Zur Anzucht adenoviraler Vektoren mussten die hergestellten adenoviralen Plasmide (nach Linearisierung durch PacI - Verdau und anschließendem Aufreinigen durch Phenol / Chloroform-Extraktion) in T-REx™-293-Zellen (Invitrogen) transfiziert werden. Dazu wurde die Methode der Calciumphosphat-Kopräzipitation verwendet, wobei die DNA über Salzkomplexe an die Zellmembran bindet und aufgenommen wird.

1 Tag vor der Transfektion wurden 800000 T-REx-Zellen aus einer logarithmisch wachsenden Kultur entnommen, mit 5 ml Medium in eine frische 25 $cm^2$-Zellkulturflasche ausgesät und über Nacht bei 37°C im Brutschrank inkubiert.

Die Zellen waren zum Zeitpunkt der Transfektion circa 50 % konfluent. 1-4 Stunden vor der Transfektion wurde das Zellkulturmedium durch frisches Medium ersetzt. Für die Transfektion wurden 15-20 μg DNA mit 169 μl sterilem $H_2O$ auf Eis vorgelegt. Dazu wurden 31 μl 2 M $CaCl_2$ pipettiert und sorgfältig vermischt. Bei Raumtemperatur wurden 250 μl 2x HBS-Puffer-Lösung tropfenweise zur $H_2O$-DNA-$CaCl_2$-Mischung gegeben. Die anschließende zehnminütige Inkubation bei Raumtemperatur ermöglichte die Ausbildung eines milchigen Präzipitats. Danach wurde der Transfektionsansatz noch einmal durchmischt und in das Zellkulturmedium getropft.

Nach maximal 18 Stunden Inkubation im Brutschrank wurde das Medium gewechselt, da das $CaCl_2$ toxisch auf die Zellen wirken kann.

In eine zweite Zellkulturflasche wurde jeweils parallel das Plasmid für AdV-eGFP (adenoviraler Vektor, der das eGFP-Protein exprimiert) transfiziert, sodass im Fluoreszenzmikroskop anhand der sich ausbildenden Fluoreszenz infizierter Zellen der Erfolg der Transfektion und der Fortschritt der Produktion von Adenoviren verfolgt werden konnte. Eine Woche später waren in der Regel alle Zellen durchlysiert, der Überstand mit den produzierten Adenoviren wurde abgenommen und konnte für die nächste Infektion zur weiteren Anzucht der adenoviralen Vektoren verwendet werden.

**Beispiel 7: Anzucht von Adenoviren**

Die adenoviralen Vektoren wurden in T-REx™-293-Zellen (Invitrogen) angezüchtet. Diese Zelllinie wurde gewählt, da sie wegen ihres Tetrazyklin-regulierten Expressionssystems zur Anzucht von Adenoviren, die ein zelltoxisches Gen, wie das Fusionsprotein von RSV exprimieren, geeignet ist. So kommt es in anderen Zelllinien bei der Adenovirusvermehrung durch die Expression des RSV-F-Proteins zur Syncytienbildung in der Zell-kultur, wodurch die Virusproduktion vermindert wird. In den T-REx-Zellen dagegen wird bei der Virusvermehrung die Expression des RSV-F-Proteins in Abwesenheit von Tetrazyklin unterdrückt, die Virusausbeute ist deutlich größer.

Die Anzucht von Adenoviren in T-REx-Zellen verlief über mehrere Infektionszyklen. Bei den ersten Passagen wurde jeweils eine 75 $cm^2$ große Zellkulturflasche infiziert, später jeweils eine 175 $cm^2$ große Zellkulturflasche. Zur Infektion wurden jeweils 3-8 ml des eine Passage zuvor geernteten Zellkulturüberstands in einer geeigneten Menge frischen Mediums verwendet. Zum Zeitpunkt der Infektion waren die Zellkulturen dabei circa 70 %ig konfluent. In den ersten vier Stunden nach Viruszugabe wurden die Zellen regelmäßig geschwenkt, um die Infektionsraten zu erhöhen. Dann erfolgte ein Mediumwechsel (10-15 ml Medium bei der 75 $cm^2$ großen Flasche, 20 ml bei der 175 $cm^2$ großen Flasche).

Der Fortschritt der Infektion wurde täglich kontrolliert und zeigte sich am CPE (cytopathischer Effekt), welcher als Abkugeln der Zellen ersichtlich ist. Die Virusernte erfolgte nach 3-6 Tagen, sobald alle Zellen durchinfiziert und abgeschwommen waren. Dabei wurde der Zellkulturüberstand abgenommen und anschließend dreimal abwechselnd bei -80°C eingefroren und im 25°C-Wasserbad aufgetaut, damit auch die Zellen, die bisher noch nicht aufgeplatzt waren, die produzierten Adenoviren freisetzten. Nach den drei Einfrier/Auftau-Zyklen wurde der Zellschrott bei 1000g und 4°C für 10 Minuten abzentrifugiert. Der Überstand mit den Adenoviren wurde bei -80°C gelagert und für die nächste Infektion verwendet.

Die adenoviralen Vektoren wurden solange in T-REx-Zellen passagiert, bis ein möglichst hoher Titer (bestimmt nach dem Verfahren der GTU) von idealerweise mindestens $10^{10}$ GTU / ml erreicht wurde. Dann wurden erst 2-3 175 $cm^2$ große Zellkulturflaschen infiziert und mit dem davon geernteten Überstand 5 oder 10 175 $cm^2$ große zellkulturflaschen, um genügend Ausgangsmaterial für die anschließende Virusaufreinigung zu erhalten.

**Beispiel 8: Aufreinigung und Konzentrierung von Adenoviren**

Die Aufreinigung und Konzentrierung der adenoviralen Vektoren erfolgte mit dem Kit Vivapure® AdenoPACK™ 100 (Sartorius) entsprechend der Anleitung des Herstellers. Dabei wurde eine Konzentrierung um den Faktor 1000 erreicht. Der Pufferaustausch-Schritt im Rahmen der Schlusakonzentrierung wurde durchgeführt, wobei die gewonnenen Adenoviren in Storage Buffer aufgenommen wurden.

**Beispiel 9: Titerbestimmung adenoviraler Präparationen**

Zur Titerbestimmung adenoviraler Vektoren wurden zwei verschiedene Methoden verwendet.

Bei der Bestimmung der OPU (optische Partikel-Einheiten) wird der Titer der adenoviralen Präparation über eine photometrische DNA-Bestimmung ermittelt. Dabei werden alle DNA-enthaltenden viralen Partikel erfasst, das heißt auch defekte Partikel, die nicht infektiös sind oder die nicht das gewünschte Gen tragen. Vorteile der OPU-Bestimmung sind, dass diese Methode der Titerbestimmung die schnellste ist und die höchste Reproduzierbarkeit aufweist.

Zur Bestimmung der OPU wurde eine Verdünnungsreihe mit beispielsweise den Verdünnungen 1:2, 1:5, 1:10, 1:20, 1:25 und 1:50 von der adenoviralen Präparation hergestellt. Dabei wurden die aufgereinigten Adenoviren jeweils in Virion Lysis Buffer (0,1 % SDS, 10 mM Tris-HCl pH 7,4, 1 mM EDTA in $H_2O$) verdünnt. Für jede Verdünnungsstufe wurde eine Blanklösung hergestellt, indem analog Storage Buffer (10 % Glycerin in Dulbecco's modified PBS) (anstatt der adenoviralen Präparation) in Virion Lysis Buffer verdünnt wurde. Nach einer Inaktivierung der Virus-haltigen Proben bei 56°C für 10 Minuten wurde die Absorption der Verdünnungen bei der Wellenlänge $\lambda$=260 nm ($OD_{260}$) gemessen, wobei das Photometer zuvor jeweils mit der zugehörigen Blanklösung eingestellt wurde. Die OPU wurde anschließend aus den $OD_{260}$-Werten nach der folgenden Formel, bei der als Extinktionskoeffizient der Wert 1,1 x $10^{12}$ [74] gewählt wird, bestimmt:

$$\text{OPU / ml} = OD_{260} \times \text{Verdünnung der Präparation} \times 1{,}1 \times 10^{12}$$

Dabei wurde die OPU als Durchschnitt der für die einzelnen Verdünnungen berechneten Werte ermittelt.

Da die OPU (wie oben beschrieben) auch defekte Partikel einschließt, wurde zusätzlich die GTU (gentransduzierende Einheiten), das heißt der Anteil der infektiösen und gentransduzierenden und somit gewünschten adenoviralen Vektoren an der OPU, anhand einer seriellen Verdünnungsreihe auf 293A-Zellen mit anschließender spezifischer immunzytochemischer Färbung des auf infizierten Zellen exprimierten RSV-F-Proteins bestimmt.

Die Titration wurde auf 293A-Zellen durchgeführt. Dazu wurde eine 96-Well-Platte Typ F mit circa 10000 293A-Zellen (in 100 µl Medium) pro Well belegt und im Brutschrank kultiviert. Einen Tag später wurde die Titerplatte infiziert. Dabei wurde die adenovirale Präparation im Verhältnis 1:1000 ins erste Well der Titerplatte gegeben (10 µl einer 1:100 Vorverdünnung wurden zu den 100 µl Medium im ersten Well hinzugegeben) und horizontal sukzessive von Well zu well seriell 1:10 weiter verdünnt (jeweils 10 µl wurden von Well zu Well transferiert). Diese Verdünnungsreihe wurde wiederholt in 3-5 Reihen durchgeführt. 2-3 Reihen belieben als Negativkontrolle Virus-frei und wurden analog verdünnt.

2-3 Tage nach der Infektion der Titerplatte wurde eine spezifische immuncytochemische Färbung des F-Proteins von RSV durchgeführt. Bei Testung des adenoviralen Vektors AdV-$F_{syn}$ (adenoviraler Vektor, der die Nukleotidsequenz von SEQ ID NO: 2 enthält) war dies direkt möglich, da die infizierten Zellen das RSV-F-Protein auf ihrer Oberfläche exprimieren.

Bei der Färbung der Titerplatte wurde im ersten Schritt das Medium abgenommen und die Zellen für 10 Minuten mit 100 µl 80 %igem Ethanol pro Well fixiert. Nach Abnehmen des Ethanols wurde die Platte an der Luft getrocknet. Dann wurden zur Rehydrierung 200 µl PBS-$T_{0.05\,\%}$ (0, 05 % (v/v) Tween 20 in PBS) in jedes Well gegeben und 5 Minuten lang auf der Platte belassen. Die Platte wurde ausgeklopft, dann wurden in jedes Well 100 µl einer 1:250 Verdünnung des 18F12-Erstantikörpers in PBS-$T_{0,05\,\%}$ gegeben. Die Platte wurde anschließend für 1 Stunde bei 37°C inkubiert. Dabei bindet der 18F12-Antikörper, der gegen das F-Protein von RSV gerichtet ist, an das auf infizierten Zellen exprimierte F-Protein. In einem dreimaligen Waschschritt mit PBS-$T_{0.05\,\%}$ wurde nicht gebundener Antikörper entfernt. Zur Detektion des Erstantikörpers wurde als Enzym-gekoppelter Zweitantikörper Kaninchen anti-Maus Immunglobulin P0260 (Dako), welches an den monoklonalen 18F12-Antikörper bindet, verwendet und 1:400 in PBS-$T_{0.05\,\%}$ verdünnt. Von dieser Lösung wurden 100 µl in jedes Well gegeben, die Platte wurde erneut für 1 Stunde bei 37°C inkubiert. Nach einem dreimaligen Waschschritt mit PBS-$T_{0,05\,\%}$ wurde die durch das am Sekundärantikörper gebundene Enzym vermittelte Farbreaktion durch Zugabe von 100 µl der FärbeLösung (200 µl AEC (3-Amino-9-ethylcarbazol 10mg/ml gelöst in 96% Ethanol) und 10 µl $H_2O_2$ in 10 ml Phosphat-Citrat) in jedes Well gestartet. Die Farbreaktion erfolgte bei einer Inkubation bei 37°C für 30 Minuten und wurde durch Zugabe von 100 µl $H_2O$ pro Well nach Ausklopfen der Platte gestoppt.

Die Interpretation erfolgte unter dem Mikroskop, indem für jede Reihe die Anzahl der gefärbten Zellen in dem letzten Well, in dem anhand der Anfärbung noch eine Infektion nachgewiesen werden konnte, bestimmt und mit der in diesem Well vorliegenden Verdünnungsstufe multipliziert wurde. Die GTU berechnet sich aus dem Durchschnitt aller Reihen.

Die oben beschriebene Färbemethode bezieht sich auf die Titerbestimmung von Präparationen RSV-F-Protein exprimierender adenoviraler Vektoren. Bei der Bestimmung des Titers einer Präparation von AdV-eGFP dagegen wurde keine Färbung durchgeführt, die Titerplatte wurde bei Betrachtung im Fluoreszenzmikroskop durch Zählung der grün leuchtenden Zellen ausgewertet.

**Beispiel 10: Immunisierungsstudie im BALB/c-Mausmodell**

Im Rahmen einer ersten Immunisierungsstudie wurde eine Gruppe von 6 BALB/c-Mäusen subkutan mit 1 x $10^8$ GTU (5 x $10^9$ OPU) AdV-$F_{syn}$ immunisiert. Ein Boost erfolgte nach 4 Wochen. Zum Vergleich wurde eine Gruppe von ebenfalls 6 Mäusen subkutan mit Plasmid-DNA immunisiert. Dabei wurde das Plasmid pcD$F_{syn\ ED}$ verwendet, welches für die Ektodomäne des synthetischen F-Proteins von RSV kodiert und in Vorversuchen mit BALB/c-Mäusen im Vergleich mit den Plasmiden pcI$F_{syn}$ und pcI$F_{wt}$, die für das synthetische F-Protein (Volllänge) beziehungsweise das Wildtyp-F-Protein von RSV kodieren, den besten Schutz nach DNA-Immunisierung vor RSV induziert hatte (Figur 1). FI-RSV diente hier als Kontrolle der Induktion der Immunantwort gegen RSV und bezeichnet die zur Immunisierung verwendeten, mit Formalin inaktivierten RS-Viren. Als Kontrollgruppen wurden 6 Mäuse subkutan mit AdV-OVA (adenoviraler Vektor, der Ovalbumin exprimiert) immunisiert, 3 Mäuse wurden überhaupt nicht immunisiert. Drei Wochen nach der zweiten Immunisierung wurden die Mäuse intranasal mit ca. 1 x $10^7$ iE (infektiöse Einheiten) von Plaque aufgereinigtem RSV infiziert.

Am Tag 5 nach Infektion wurden die Mäuse nach Betäubung getötet und eine BAL (bronchoalveoläre Lavage) gewonnen. Aus der BAL wurde die virale RNA extrahiert und als Maß für den Schutz der Tiere der RSV-RNA-Gehalt mittels quantitativer RT-PCR bestimmt. Bei den Kontrollgruppen wurde mit durchschnittlich circa 20000 RSV-Kopien eine hohe RSV-Beladung bestimmt. Während nach Immunisierung mit pcD$F_{syn}$ ED die RSV-Kopienzahl bereits deutlich um einen Faktor von circa 55 (durchschnittlich 360 Kopien pro BAL) reduziert war, war nach Immunisierung mit AdV-$F_{syn}$ bei allen Mäusen der Gruppe überhaupt kein RSV mehr nachweisbar, das heißt, die RSV-Beladung wurde hier um einen Faktor von mindestens etwa 400 reduziert, da die Nachweisgrenze bei der qRT-PCR bei 50 Kopien liegt. Somit sind die Tiere nach Impfung mit pcD$F_{syn\ ED}$ nur bedingt vor RSV geschützt, da die RSV-Replikation in den Lungen der Mäuse nur gehemmt, nicht aber vollständig verhindert wird. Nach Impfung mit AdV-$F_{syn}$ dagegen scheinen die Tiere vollständig geschützt zu sein, da kein RSV mehr nachweisbar ist (Figur 2).

Im Verlauf des Versuchs wurde den Mäusen in der Woche 0 (vor der ersten Immunisierung) und 7 (nach der zweiten Immunisierung) sowie am Tötungstag Blut entnommen und davon Serum gewonnen. Eine zusätzliche Blutentnahme nach der ersten und vor der zweiten Immunisierung wurde in dieser ersten Immunisierungsstudie noch nicht durchgeführt.

Mittels der Serumproben wurde im Antikörper-ELISA der Titer an RSV-spezifischen IgG1- und IgG2a-Antikörpern im Blut der Mäuse bestimmt. Da eine Standardisierung dieses ELISAs bisher noch nicht möglich war, können die Ergebnisse hierbei nur innerhalb der Gruppen einer Immunisierungsstudie verglichen werden. Dabei werden alle Proben einer Immunisierungsstudie zeitgleich auf einer Elisa-Platte untersucht.

Vor Immunisierung konnten bei keiner Mäusegruppe RSV-spezifische IgG1- oder IgG2a-Antikörper nachgewiesen werden. In den Kontrollgruppen blieb der Titer im Verlauf des Experiments gleich niedrig. Bei den mit AdV-$F_{syn}$ oder mit $pcDF_{synED}$ geimpften Mäusen dagegen konnte nach der zweiten Immunisierung und am Tötungstag ein klarer Anstieg der RSV-spezifischen Antikörper gezeigt werden. Während die IgGl-Antikörper bei beiden Gruppen auf ähnliche Werte anstiegen, war die Erhöhung der IgG2a-Antikörper nach Impfung mit AdV-$F_{syn}$ vielfach stärker als nach Impfung mit $pcDF_{synED.}$ Somit bewirkt die zweifache Immunisierung mit AdV-$F_{syn}$ eine starke Immunantwort hinsichtlich der Bildung systemischer RSV-spezifischer IgG-Antikörper und induziert hierbei die Herstellung von weitaus mehr IgG2a als nach zweifacher Immunisierung mit $pcDF_{synED}$ (Figur 3). Dass bei den mit AdV-$F_{syn}$ geimpften Mäusen die IgG2a-Antikörper vielfach stärker als die IgG1-Antikörper ansteigen, deutet auf eine $T_H1$-basierte Immunantwort nach Immunisierung mit AdV-$F_{syn}$ hin, was als besonders positiv beurteilt werden kann, da die in den 60er Jahren nach Immunisierungsversuchen mit Formalininaktiviertem RSV beobachtete Krankheitsverschlimmerung vor allem auf eine Verschiebung der Immunantwort zugunsten einer $T_H2$-Antwort zurückgeführt wurde.

Die Seren der Mäuse wurden außerdem im Neutralisationstest auf neutralisierende Antikörper gegen RSV getestet. Dabei waren zu Beginn des Versuchs in keiner Mäusegruppe neutralisierende Antikörper vorhanden. Während in den Kontrollgruppen bei allen Serumproben keine neutralisierenden Antikörper gegen RSV gefunden werden konnten, führte die Immunisierung mit AdV-$F_{syn}$ oder $pcDF_{synED}$ zur Bildung neutralisierender Antikörper. Dabei stiegen diese nach Adv-$F_{syn}$-Impfung ungefähr 13fach stärker an als nach Impfung mit $pcDF_{synED}$, sodass die Immunisierung mit AdV-$F_{syn}$ auch hier als der Impfung mit $pcDF_{synED}$ überlegen angesehen werden kann. Im Vergleich zu den Serumproben nach der zweiten Immunisierung fand sich bei den Serumproben vom Tötungstag, das heißt nach Challenge, jeweils eine leichte Verminderung des Titers neutralisierender Antikörper, was durch einen Verbrauch dieser im verlauf der Infektion erklärt werden kann (Figur 4).

In den durchgeführten Immunisierungsstudien konnte demnach die Überlegenheit von AdV-$F_{syn}$ über $pcDF_{synED}$ deutlich gezeigt werden (Induktion eines höheren Titers an RSV-spezifischen Antikörpern, geringere RSV-Beladung in der Lunge und entsprechend besserer Schutz der Mäuse).

**Beispiel 11: Immunisierung mit AdV-$F_{syn}$ über verschiedene Administrationsrouten**

In diesem Experiment sollte der Einfluss der Administrationsroute auf die Immunisierung mit AdV-Fsyn untersucht werden. Dazu wurden drei Gruppen von je 6 BALB/c-Mäusen mit $1 \times 10^8$ GTU ($5 \times 10^9$ OPU) AdV-$F_{syn}$ immunisiert, wobei der Impfstoff bei der ersten Gruppe intranasal, bei der zweiten intramuskulär und bei der dritten subkutan verabreicht wurde. Eine vierte Gruppe mit ebenfalls 6 BALB/c-Mäusen wurde als Negativkontrolle nicht immunisiert. Das Zeitschema für Impfungen, Blutentnahmen und Challenge wurde von der ersten Immunisierungsstudie übernommen (siehe Beispiel 10).

Am Tötungstag (5 Tage nach Infektion) wurde die BAL der Mäuse gewonnen. Nach Isolierung viraler RNA aus der BAL wurde wieder der RSV-RNA-Gehalt mittels quantitativer RT-PCR bestimmt, um eine Aussage über den Schutz der Tiere nach Impfung treffen zu können. Während die RSV-Beladung in der nicht-immunisierten Kontrollgruppe sehr hoch war, wiesen alle drei mit AdV-$F_{syn}$ geimpften Mäusegruppen einen sehr guten Schutz auf. Abgesehen von einer einzigen Maus aus der intramuskulär geimpften Mäusegruppe, bei der die RSV-Beladung in der BAL bei 88 Kopien lag, war in keiner BAL eine RSV-Beladung nachweisbar, das heißt, dass alle Mäuse unabhängig von der Administrationsroute nach Impfung mit AdV-$F_{syn}$ sehr gut geschützt waren (Reduktion um einen Faktor von mindestens 160 in allen drei Mäusegruppen) (Figur 5).

Da bei der quantitativen RT-PCR, die anhand der RNA-Isolate aus den BALs der Mäuse durchgeführt wurde, keine Unterschiede bezüglich der RSV-Beladung in den Lungen der verschiedenen Mäusegruppen festgestellt werden konnten, wurde zusätzlich die Gesamt-RNA aus dem Lungenhomogenat, welches ebenfalls am Tötungstag gewonnen worden war, isoliert. Diese RNA-Isolate wurden ebenfalls in der quantitativen RT-PCR auf ihren RSV-Gehalt getestet, um einen zweiten Wert für die RSV-Beladung in den Mäuselungen und somit für den Schutz der Mäuse vor RSV zu erhalten. Dabei wurden die Ergebnisse der qRT-PCR anschließend in Bezug auf den RNA-Gehalt des RNA-Isolats umgerechnet, da bei der RNA-Isolierung nicht nur virale RNA, sondern die Gesamt-RNA isoliert wurde. Bei der quantitativen RT-PCR wurde so in der nicht-immunisierten Kontrollgruppe eine RSV-Kopienzahl von durchschnittlich circa 160000 Kopien / µg RNA bestimmt. Die Bestimmung der RSV-Beladung anhand von RNA-Isolaten aus dem Lungenhomogenat ist somit wesentlich sensitiver als die anhand von RNA-Isolaten aus der BAL, wo in der nicht-immunisierten Kontrollgruppe nur eine RSV-Beladung von durchschnittlich circa 8000 Kopien / BAL gefunden wurde. Dementsprechend zeigten sich hier auch Unterschiede in den immunisierten Mäusegruppen. Der beste Schutz wurde nach intranasaler Immunisierung mit AdV-$F_{syn}$ erreicht. Hier lag die RSV-Beladung in der qRT-PCR bei allen Mäusen unterhalb der Nachweisgrenze von 50 Kopien. Umgerechnet in Bezug zum RNA-Gehalt der Proben heißt das, dass die intranasal geimpften Tiere nur eine sehr geringe RNA-Beladung aufwiesen. Sogar unter Annahme von 50 Kopien als Ergebnis der qRT-PCR war 1 Tier komplett geschützt, während die anderen 5 Tiere 1-5 Kopien / µg RNA aufwiesen. Somit wird durch die intranasale Immunisierung ein sehr guter, nahezu kompletter Schutz vor RSV induziert, wobei die RSV-Beladung um einen Faktor von circa 70000 reduziert wird. Nach intramuskulärer und subkutaner Impfung mit AdV-$F_{syn}$ dagegen war eine RSV-Beladung in der qRT-PCR nachweisbar. Pro Gruppe waren nur jeweils 2 Tiere mit einer RSV-Beladung von 2 Kopien / µg RNA sehr gut geschützt, die jeweils anderen 4 Tiere zeigten eine durchschnittliche RSV-Beladung von circa 570 Kopien / µg RNA nach intramuskulärer Impfung (circa 280fache Reduktion) beziehungsweise circa 275 Kopien / µg RNA nach subkutaner Impfung (circa 580fache Reduktion) (Figur 6). Somit ist die intranasale Route bei einer Immunisierung mit AdV-$F_{syn}$ eindeutig am besten geeignet, um einen sehr guten Schutz vor RSV zu induzieren.

Die im Verlauf des Experiments gewonnenen Seren der Mäuse wurden wieder im IgG-Antikörper-ELISA und im Neutralisationstest auf die Induktion systemischer RSV-spezifischer Antikörper getestet.

Vor der Immunisierung waren in keiner Mäusegruppe RSV-spezifische IgG1- oder IgG2a-Antikörper im ELISA nachweisbar. In der Kontrollgruppe blieb der Titer auch bei den weiteren Serumproben ähnlich niedrig. Bei den drei mit AdV-$F_{syn}$ geimpften Mäusegruppen dagegen fand sich ein deutlicher RSV-spezifischer Titeranstieg sowohl bei IgG1- als auch bei IgG2a-Antikörpern. Dabei war dieser Anstieg von der Administrationsroute von AdV-$F_{syn}$ abhängig, da der Antikörpertiter in den intranasal (i.n.), intramuskulär (i.m.) und subkutan (s.c.) geimpften Mäusen jeweils unterschiedlich ausfiel. So variierte der Titer vor allem der IgG2a-Antikörper recht stark, die subkutane Immunisierung führte zu einem vielfach stärkeren IgG2a-Antikörperanstieg als die intranasale oder die intramuskuläre Impfung. Die Unterschiede im IgG1-Titer waren dagegen nicht so stark ausgeprägt, da die Induktion von IgG1-Antikörpern bei allen Mausgruppen schwächer ausfiel als die der IgG2a-Antikörper. Trotzdem ist ersichtlich, dass hier die subkutane Immunisierung den schwächsten IgG1-Anstieg bewirkte. Bereits im Rahmen der ersten Immunisierungsstudie konnte gezeigt werden, dass die subkutane AdV-$F_{syn}$-Impfung einen vielfach höheren IgG2a- als IgG1-Antikörper-Titer induziert, was für eine $T_H$1-vermittelte Immunreaktion spricht. Dieses Ergebnis zeigte sich auch hier im zweiten Versuch bei der subkutanen Impfung. Bei intramuskulärer und intranasaler Immunisierung mit AdV-$F_{syn}$ dagegen war die IgG2a / IgG1-Ratio kleiner. Während nach intramuskulärer Impfung noch mehr IgG2a- als IgG1-Antikörper gebildet wurden, war das Verhältnis von IgG2a und IgG1 nach intranasaler Immunisierung recht ausgeglichen (Figur 7). Somit wird der $T_H$1-vermittelte Weg bei der Immunreaktion gegen RSV am stärksten bei der subkutanen Immunisierung angesprochen. Aber auch bei intranasaler Impfung ist das Verhältnis von $T_H$1 (IgG2a) und $T_H$2 (IgG1) ausgeglichen, was für eine balancierte $T_H$1 / $T_H$2-Antwort spricht. Somit wird der IgG-Antikörperklassen-Verteilung zufolge über keine der drei Administrationsrouten eine überschießende $T_H$2-Antwort erzeugt, was für eine Impfung gegen RSV bedeutsam ist.

Des Weiteren war bei allen drei mit AdV-$F_{syn}$ geimpften Mäusegruppen von der Serumprobe nach erster Immunisierung zur Serumprobe nach zweiter Immunisierung bei beiden IgG-Antikörperklassen ein weiterer Titeranstieg vorhanden, wobei dieser bei intranasaler Immunisierung am geringsten und bei subkutaner Immunisierung am stärksten ausgeprägt war (Figur 7). Man kann daher feststellen, dass ein Boost bei AdV-$F_{syn}$-Impfung einen weiteren Antikörperanstieg induziert und somit für den Aufbau einer guten Immunität gegen RSV sinnvoll ist.

Auch im Neutralisationstest zeigte sich wieder, dass die Impfung mit AdV-$F_{syn}$ eine sehr starke spezifische Serum-Antikörper-Antwort gegen RSV induziert: Während vor der ersten Immunisierung in allen Mäusegruppen keine neutralisierenden Antikörper gegen RSV nachgewiesen werden konnten und dieser Titer in der nicht-immunisierten Kontrollgruppe auch nicht anstieg, zeigte sich bei den drei mit AdV-$F_{syn}$ geimpften Gruppen eine starke Erhöhung RSV-spezifischer neutralisierender Antikörper. Wie im IgG-ELISA und bei der RSV-qRT-PCR (Lungenhomogenat) waren auch hier Unterschiede abhängig von der Administrationsroute vorhanden. So führte die intranasale Immunisierung zur stärksten Erhöhung der neutralisierenden Aktivität, wobei der Titer um einen Faktor von circa 200 anstieg. Dabei war der Titeranstieg nach intranasaler Immunisierung 10fach stärker als nach intramuskulärer Impfung (hier fand sich ein Titeranstieg um einen Faktor von circa 20) und 6fach stärker als nach subkutaner Impfung (hier fand sich ein Titeranstieg um einen Faktor von circa 35). Somit wurde gezeigt, dass die Immunisierung mit AdV-$F_{syn}$ unabhängig von der Administrationsroute (intranasal, intramuskulär, subkutan) eine sehr gute Antwort an systemischen neutralisierenden Antikörpern gegen RSV hervorruft, wobei die intranasale Route die stärkste Antwort induziert (Figur 8).

Weiterhin zeigte sich hier, wie auch schon im ELISA, dass die zweite Immunisierung einen weiteren Antikörperanstieg induziert. Die neutralisierende Aktivität war beim Serum nach zweiter Immunisierung im Vergleich zum Serum nach erster Immunisierung deutlich angestiegen, am stärksten bei der intranasal immunisierten Gruppe (etwa 14fach), am schwächsten bei der subkutan immunisierten Gruppe (etwa 2fach) (Figur 8).

Insgesamt fanden sich bei dem Ergebnis des Neutralisationstests stärkere Analogien zur qRT-PCR von den RNA-Isolaten aus dem Lungenhomogenat als zum ELISA. So korrespondierte der induzierte Titer an RSV-spezifischen neutralisierenden Antikörpern mit der Reduktion der RSV-Beladung in der Lunge, aber nicht mit dem Titer der im ELISA detektierten RSV-spezifischen Antikörper. Dies führt zu dem Schluss, dass der ELISA nicht nur die RSV-spezifischen neutralisierenden Antikörper detektiert, sondern vielmehr andere nicht-neutralisierende, gegen RSV gerichtete Antikörper. Da - wie aus dem Zusammenhang zwischen dem Titer an neutralisierenden Antikörpern und der Reduktion der RSV-Beladung in der Lunge ersichtlich ist - die RSV-spezifischen neutralisierenden Antikörper wesentlich an der Induktion des Schutzes vor RSV beteiligt sind, kann der Neutralisationstest besser als der ELISA zur Beurteilung des Schutzes nach Impfung herangezogen werden.

Bei der durchgeführten Immunisierungsstudie wurde mit der intranasalen Immunisierung das eindeutig beste Impfergebnis erzielt, da nach intranasaler Immunisierung mit AdV-$F_{syn}$ einerseits der höchste Titer an neutralisierenden Antikörpern gefunden wurde und andererseits die RSV-Beladung in der Lunge am stärksten (nahezu vollständig) reduziert war. Somit ist bei Prime-Boost-Vakzinierung mit AdV-$F_{syn}$ die intranasale Route zu favorisieren.

### Beispiel 12: Subkutane Immunisierung mit AdV-$Ps_{yn}$ in verschiedenen Dosierungen (Dosiseskalation)

In diesem Experiment sollte der Einfluss der Impfstoffdosis auf die subkutane Immunisierung mit AdV-$F_{syn}$ untersucht werden. Dazu wurden vier Gruppen von je 6 BALB/c-Mäusen jeweils subkutan mit Dosierungen von 2 x $10^5$ bis 2 x $10^8$ GTU (1 x $10^7$ bis 1 x $10^{10}$ OPU) AdV-$F_{syn}$ immunisiert. Eine fünfte Gruppe mit ebenfalls 6 BALB/c-Mäusen wurde nicht immunisiert (Negativkontrolle). Das Zeitschema für Impfungen, Blutentnahmen und Challenge wurde von der ersten Immunisierungsstudie übernommen (siehe Beispiel 10).

Am Tötungstag (5 Tage nach Infektion) wurden BALs und Lungenhomogenate der Mäuse gewonnen. Nach Isolierung viraler RNA aus der BAL und Gesamt-RNA aus dem Lungenhomogenat wurden quantitative RT-PCRs durchgeführt, um die RSV-Beladung in den RNA-Isolaten zu bestimmen und eine Aussage über den Schutz der Tiere nach Impfung treffen zu können. Sowohl bei den RNA-Isolaten aus den BALs als auch bei den RNA-Isolaten aus den Lungenhomogenaten war die RSV-Beladung in der nicht-immunisierten Kontrollgruppe sehr hoch, wobei die Detektion der RSV-Kopien erneut in den RNA-Isolaten aus den Lungenhomogenaten deutlich sensitiver war.

Alle 4 immunisierten Mäusegruppen wiesen bei der qRT-PCR von den Isolaten aus den BALs einen guten Schutz auf, da bei keiner einzigen Maus eine RSV-Beladung nachweisbar war (Reduktion um einen Faktor von mindestens circa 95-fach) (Figur 9).

Bei der sensitiveren qRT-PCR von den Isolaten aus den Lungenhomogenaten dagegen war auch bei den immunisierten Mäusen eine RSV-Beladung nachweisbar, wobei die RSV-Kopienzahl bei allen vier Immunisierungsgruppen vergleichbar war. So fand sich in allen Immunisierungsgruppen eine durchschnittliche Reduktion der RSV-Beladung um einen Faktor von circa 340 von durchschnittlich rund 47000 Kopien auf durchschnittlich rund 140 Kopien (Figur 10). Somit waren alle immunisierten Mäuse unabhängig von der Impfstoffdosis gleichermaßen geschützt, wobei zwar ein guter, aber nicht vollständiger Schutz zu finden war. Das bedeutet, dass der durch eine subkutane AdV-$F_{syn}$-Impfung induzierte Schutz begrenzt ist und durch eine Erhöhung der Impfstoffdosis nicht weiter gesteigert werden kann. Einerseits ist demnach bei subkutaner Impfung zum Aufbau eines guten Schutzes eine niedrige Impfstoffdosis genauso wirksam wie eine hohe Impfstoffdosis, andererseits ist die subkutane Route bei der AdV-$F_{syn}$-Impfung nicht optimal, da hier (anders als bei der intranasalen Impfung im vorherigen Experiment) ein nur begrenzter Schutz induziert werden kann.

Die im Verlauf der Immunisierungsstudie gewonnenen Seren der Mäuse wurden im IgG-Antikörper-ELISA und im Neutralisationstest auf die Induktion systemischer RSV-spezifischer Antikörper getestet.

Vor der Immunisierung waren in keiner Mäusegruppe RSV-spezifische IgG1- oder IgG2a-Antikörper im ELISA nachweisbar. In der nicht-immunisierten Kontrollgruppe fand auch im Verlauf des Experiments kein Titeranstieg statt. Bei den vier subkutan mit AdV-$F_{syn}$ geimpften Mäusegruppen dagegen fand sich ein deutlicher RSV-spezifischer Titeranstieg sowohl bei IgG1-als auch bei IgG2a-Antikörpern. Dabei war dieser Anstieg - anders als bei der RSV-qRT-PCR - von der Dosis des Impfstoffs abhängig. Bei den IgG1-Antikörpern fand sich nur ein geringer Unterschied in den Mäusegruppen, da - wie auch bereits in den bisherigen Immunisierungsstudien gezeigt wurde - bei der subkutanen Impfung grundsätzlich nur geringe IgG1-Antikörpertiter induziert werden, weil die Immunantwort $T_H1$-vermittelt verläuft. So fiel der Titeranstieg der RSV-spezifischen IgG1-Antikörper in allen vier Gruppen gleichermaßen schwach aus. Nur bei sehr hoher Impfstoffdosis konnte durch den Boost ein geringfügig stärkerer Anstieg induziert werden. Bei den IgG2a-Antikörpern dagegen wiesen die vier Mäusegruppen abhängig von der Impfstoffdosis einen deutlich variierenden Titeranstieg auf, sodass also hier eine direkte Abhängigkeit zwischen Impfstoffdosis und Titeranstieg besteht. So stieg sowohl nach der ersten Immunisierung als auch nach der zweiten Immunisierung der Titer abhängig von der Dosis unterschiedlich stark an. Während nach der ersten Immunisierung der Titer von einer Stufe der Dosierung zur nächsten (von Mäusegruppe zu Mäusegruppe) noch relativ linear, das heißt um jeweils ungefähr die gleiche Menge anstieg, zeigte sich nach dem Boost eine exponentielle Abhängigkeit, da die zweite Immunisierung bei geringer Dosis einen nur schwachen weiteren Titeranstieg bewirkte, bei hoher Dosis dagegen einen starken weiteren Titeranstieg. Somit führt die subkutane Immunisierung mit AdV-$F_{syn}$ zu einem - anders als bei der RSV-Beladung entsprechend der qRT-PCR - Dosis-abhängigen Titeranstieg RSV-spezifischer Antikörper.

Während bei hohen Dosierungen die zweite Immunisierung zu einem starken weiteren Titeranstieg führte, wurde bei geringen Dosierungen durch den Boost kaum ein weiterer Titeranstieg bewirkt, sodass - was den RSV-spezifischen Antikörpertiter entsprechend des ELISAs betrifft - der Boost nur bei den beiden höchsten Dosierungen sinnvoll scheint (Figur 11).

Es wurde auch ein Neutralisationstest auf RSV-spezifische neutralisierende Antikörper durchgeführt. Vor Immunisierung und in der nicht-immunisierten Kontrollgruppe war jeweils kein Titer nachweisbar. Nach subkutaner Impfung mit AdV-$F_{syn}$ dagegen wurden neutralisierende Antikörper gebildet. Dabei zeigte sich im Neutralisationstest ein anderes Bild als im ELISA. Hier sah man bereits nach der ersten Immunisierung eine klare Dosisabhängigkeit bei der Induktion neutralisierender Antikörper, wobei der Titer von Dosisstufe zu Dosisstufe exponentiell anstieg. So stieg der Titer bei der Dosis 2 x $10^5$ GTU (1 x $10^7$ OPU) um einen Faktor von circa 2,6, bei der Dosis 2 x $10^6$ GTU (1 x $10^8$ OPU) um einen Faktor von circa 3,3, bei der Dosis 2 x $10^7$ GTU (1 x $10^9$ OPU) um einen Faktor von circa 21, bei der Dosis 2 x $10^8$ GTU (1 x $10^{10}$ OPU) um einen Faktor von circa 36 an.

Während also im ELISA nach der ersten Immunisierung nur ein geringer Effekt der Dosiserhöhung zu beobachten war, wurden die neutralisierenden Antikörper durch eine hohe Impfstoffdosis wesentlich stärker induziert als durch eine niedrige Impfstoffdosis. Nach der zweiten Immunisierung dagegen war im Neutralisationstest (anders als im ELISA) keine Dosisabhängigkeit mehr zu sehen. Der Antikörpertiter stieg unabhängig von der Impfstoffdosis nach der zweiten Immunisierung in allen immunisierten Gruppen auf einen Maximalwert, wobei jeweils die höchste Serumverdünnung, bei der die Infektion durch RSV durch neutralisierende Antikörper zu 50% (IC50) gehemmt wird, bei durchschnittlich 1:295 lag. Das heißt, dass bei der zweifachen subkutanen Immunisierung mit AdV-$F_{syn}$ nur ein maximaler Titer an neutralisierenden Antikörpern aufgebaut und dieser auch nicht durch eine weitere Dosiserhöhung gesteigert werden kann. Dieses Ergebnis deckt sich mit dem der qRT-PCR von RNA-Isolaten aus dem Lungenhomogenat, da auch hier bei allen immunisierten Mäusegruppen unabhängig von der Impfstoffdosis ein vergleichbar guter Schutz (ähnliche Reduktion der RSV-Beladung) gefunden wurde, was erneut zeigt, dass die im Neutralisationstest detektierten Antikörper wesentlich für den Schutz vor RSV-Infektion verantwortlich sind. Somit zeigt das Ergebnis des Neutralisationstests genauso wie die RSV-qRT-PCR, dass ein guter Schutz vor RSV-Infektion bei zweifacher subkutaner AdV-$F_{syn}$-Immunisierung auch durch eine niedrige Impfstoffdosis erreicht und durch Dosiserhöhung nicht weiter gesteigert werden kann. Wie aus dem Neutralisationstest ersichtlich ist, scheint dabei aber der Boost unbedingt nötig zu sein, um durch eine niedrige Impfstoffdosis einen genauso guten Schutz wie durch eine hohe Impfstoffdosis zu induzieren (Figur 12).

Bei der zweifachen subkutanen Immunisierung mit AdV-$F_{syn}$ wird demnach nur ein begrenzter Schutz erzielt, da der maximal erreichte Wert bezüglich Reduktion von RSV-Beladung in der Lunge und induziertem Titer an neutralisierenden Antikörpern auch durch Dosiserhöhung nicht weiter gesteigert werden kann. Dieses Ergebnis verstärkt den aus der vorherigen Immunisierungsstudie gezogenen Schluss, dass die intranasale Route zu favorisieren ist. Des Weiteren scheint bei der subkutanen Immunisierung mit AdV-$F_{syn}$, da nach der zweiten Immunisierung in allen Mäusegruppen der gleiche maximale Titer an neutralisierenden Antikörpern erreicht und die RSV-Beladung in der Lunge gleichermaßen reduziert wurde, für den gleichen Impferfolg auch eine geringere AdV-$F_{syn}$-Dosis ausreichend zu sein, sofern eine Prime-Boost-Strategie gewählt wird.

**Beispiel 13: Expresionnteigerung von RSV-F Protein bei der Verwendung codon-optimierter Expressionssequenzen.**

Nach Einbringen (Transfektion) der Expressionsplasmide pFwt (Expressionsplasmid mit der Wildtyp Sequenz des RSV-F Proteins), pIFwt (Expressionsplasmid mit der Wildtyp Sequenz des RSV-F Proteins mit einem zusätzlichen Intron vor dem offenen Leserahmen), pFsyn (Expressionsplasmid mit der codon-optimierten Sequenz des RSV-F Proteins) und pIFsyn (Expressionsplasmid mit der codon-optimierten Sequenz des RSV-F Proteins mit einem zusätzlichen Intron vor dem offenen Leserahmen) in Hep2 Zellen wurden diese 48 Stunden danach lysiert. Die Proteine des Zelllysats wurden mittels Gelelektrophorese der Größe nach aufgetrennt und anschließend auf eine Nitrocellulosemembran transferriert.

In den Zelllysaten wurde die Menge an exprimiertem RSV-F Protein bestimmt (Figur 14). Als Kontrollen dienten ein Plasmid ohne Fremdgen (Leerplasmid; pcDNA3.1), Zellen ohne Plasmid (Hep2) und mit RSV infizierte Zellen (Positivkontrolle; RSV). Die Ergebnisse zeigen, dass nur durch die Codon-Optimierung eine Expression erreichbar ist (Vergleich von pFwt oder pIFwt zu pFsyn oder pIFsyn).

Die Expression des codon-optimierten Konstrukts (pIFsyn) wurde durch serielle Verdünnung (1:$10^2$ bis 1:$10^4$) des Zelllysates mit der Expression des Plasmids mit der ursprünglichen Sequenz (pIFwt) im unverdünnten Zelllysat (1: 1) verglichen (Figur 15). Die Proteinmenge von RSV-F durch Expression des codon-optimierten Konstrukts liegt bei einer Verdünnung von 1000-fach (1:$10^3$) noch deutlich über der durch das Plasmid mit der ursprünglichen Sequenz. (kDa = Molekulargewicht in Kilodalton)

Die Ergebnisse zeigen, dass die erfindungsgemäße, codon-optimierte Nukleinsäuresequenz mit der SEQ ID NO:2 die Expression des RSV-F Proteins in Zellen ungewöhnlich stark steigert (Figur 14; Figur 15). Im Gegensatz zur bekannten Expressionssteigerungen von in der Regel 50 bis höchstens 100-fach, wird im Fall der Verwendung der erfindungsgemäßen Sequenz eine Steigerung der Expression von RSV F Protein von über 1000-fach gefunden. Im Gegensatz zu anderen viralen Proteinen, z.B. VSV-G Protein, lässt sich bei dem RSV-F Protein allein durch die Vorschaltung eines Introns keine Expressionsverstärkung erzielen.

## Patentansprüche

1. Nukleinsäuremolekül umfassend eine Nukleotidsequenz, die für ein F-Protein des Respiratorischen Syncytialvirus (RSV) kodiert, **dadurch gekennzeichnet, dass** die Nukleotidsequenz SEQ ID NO: 2 umfasst

2. Vektor, **dadurch gekennzeichnet, dass** der Vektor das Nukleinsäuremolekül gemäß Anspruch 1 umfasst.

3. Vektor nach Ansprüch 2, wobei der Vektor ein viraler Vektor oder ein Plasmid ist.

4. Vektor nach Anspruch 3, **dadurch gekennzeichnet, dass** der virale Vektor ein adenoviraler Vektor ist, der (a) ein Nukleinsäuremolekül gemäß Anspruch 1 und (b) eine Transkriptions- und/oder Translationskontrollsequenz in funktionaler Verknüpfung mit dem Nukleinsäuremolekül umfasst.

5. Zelle, die einen Vektor nach einem der Ansprüche 2-4 umfasst.

6. Immunogene Zusammensetzung, **dadurch gekennzeichnet, dass** die Zusammensetzung ein Nukleinsäuremolekül gemäß Anspruch 1 umfasst oder einen Vektor gemäß einem der Ansprüche 2-4 und einen pharmazeutisch annehmbaren Träger umfasst.

7. Verwendung eines Nukleinsäuremoleküle gemäß Anspruch 1 oder eines Vektor gemäß einem der Ansprüche 2-4 für die Herstellung eines Impfstoffes zur Impfung eines Subjekts gegen durch eine RSV-Infektion verursachte Erkrankung.

8. Verfahren zur Herstellung eines RSV F-Proteins, **dadurch gekennzeichnet, dass** das Verfahren (a) die Transfektion einer Wirtszelle mit einem Nukleinsäuremolekül gemäß Anspruch 1 und (b) die Expression des Nukleinsäuremoteküls in der Wirtszelle umfasst.

## Claims

1. A nucleic acid molecule comprising a nucleotide sequence which encodes for an F-protein of the respiratory syncytial virus (RSV), **characterized in that** the nucleotide sequence comprises SEQ ID NO: 2.

2. A vector, **characterized in that** the vector comprises the nucleic acid molecule according to claim 1.

3. The vector according to claim 2, wherein the vector is a viral vector or a plasmid.

4. The vector according to claim 3, **characterized in that** the viral vector is an adenoviral vector which comprises (a) a nucleic acid molecule according to claim 1, and (b) a transcription control sequence and/or translation control sequence in functional linkage with the nucleic acid molecule.

5. A cell which comprises a vector according to one of the claims 2-4.

6. An immunogenic composition, **characterized in that** the composition comprises a nucleic acid molecule according to claim 1 or a vector according to one of the claims 2-4 and a pharmaceutical acceptable carrier.

7. Use of a nucleic acid molecule according to claim 1 or of a vector according to one of the claims 2-4 for the preparation of a vaccine for the vaccination of a subject against disease caused by an RSV-infection.

8. A method for the preparation of an RSV F-protein, **characterized in that** the method comprises (a) the transfection of a host cell with a nucleic acid molecule according to claim 1 and (b) the expression of the nucleic acid molecule in the host cell.

**Revendications**

1. Molécule d'acide nucléique comprenant une séquence de nucléotides qui code pour une protéine F du virus respiratoire syncytial (RSV), **caractérisée en ce que** la séquence de nucléotides comprend SEQ ID N° : 2.

2. Vecteur, **caractérisé en ce que** le vecteur comprend la molécule d'acide nucléique selon la revendication 1.

3. Vecteur selon la revendication 2, le vecteur étant un vecteur viral ou un plasmide.

4. Vecteur selon la revendication 3, **caractérisé en ce que** le vecteur viral est un vecteur adénoviral qui comprend (a) une molécule d'acide nucléique selon la revendication 1 et (b) une séquence de contrôle de la transcription et/ou de la traduction reliée fonctionnellement à la molécule d'acide nucléique.

5. Cellule qui comprend un vecteur selon l'une des revendications 2 à 4.

6. Composition immunogène, **caractérisée en ce que** la composition comprend une molécule d'acide nucléique selon la revendication 1, ou un vecteur selon l'une des revendications 2 à 4 et un véhicule pharmaceutiquement acceptable.

7. Utilisation d'une molécule d'acide nucléique selon la revendication 1 ou d'un vecteur selon l'une des revendications 2 à 4, pour la production d'un vaccin destiné à vacciner un sujet contre une maladie provoquée par une infection à RSV.

8. Procédé de production d'une protéine F de RSV, **caractérisé en ce que** le procédé comprend (a) la transfection d'une cellule hôte avec une molécule d'acide nucléique selon la revendication 1, et (b) l'expression de la molécule d'acide nucléique dans la cellule hôte.

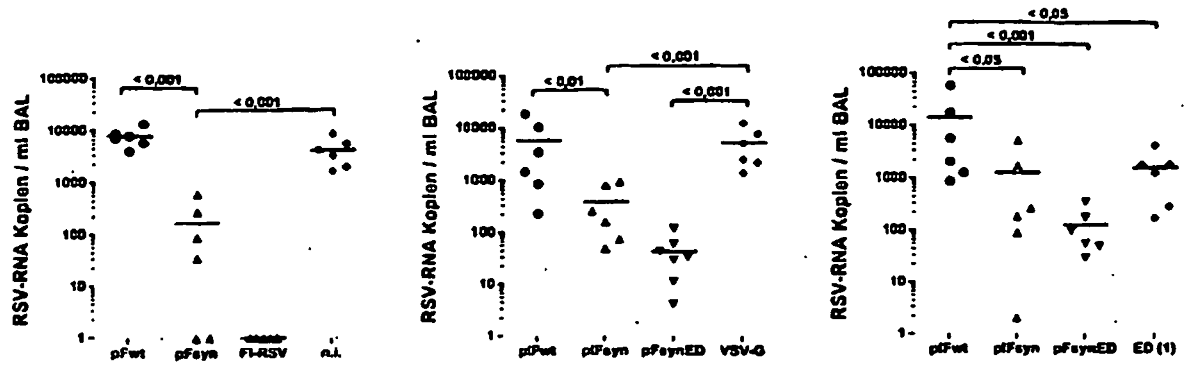
RSV-RNA Kopien / ml BAL
pFwt
pFsyn
FI-RSV
n.i.
pFsynED
VSV-G
ED (1)
< 0,001
< 0,01
< 0,05

Figur 1

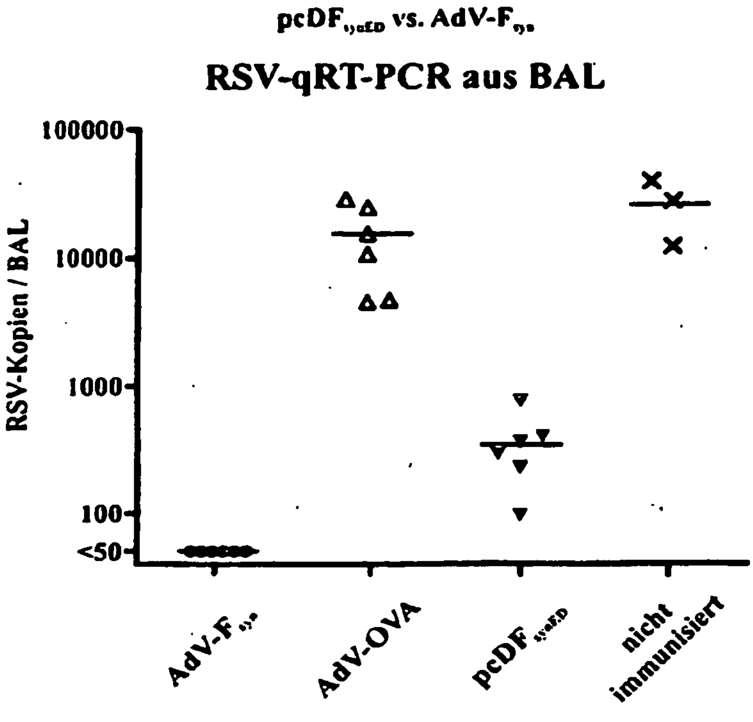
pcDF vs. AdV-F
RSV-qRT-PCR aus BAL
RSV-Kopien / BAL
100000
10000
1000
100
<50
AdV-F
AdV-OVA
pcDF
nicht immunisiert

Figur 2

pcDF$_{synED}$ vs. AdV-F$_{syn}$

IgG1-ELISA

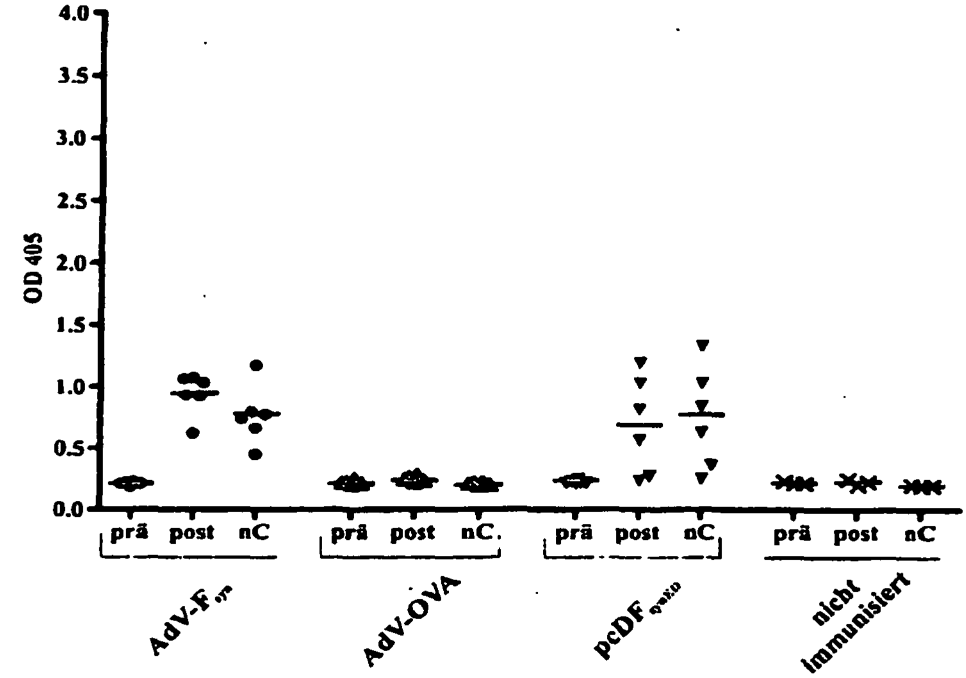


pcDF$_{synED}$ vs. AdV-F$_{syn}$

IgG2a-ELISA

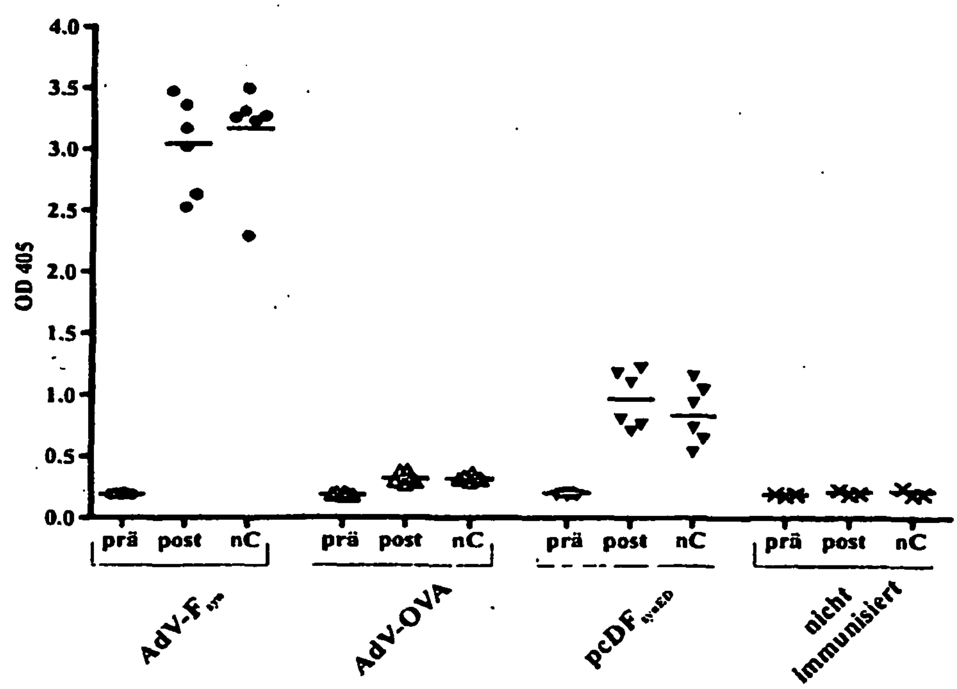


Figur 3

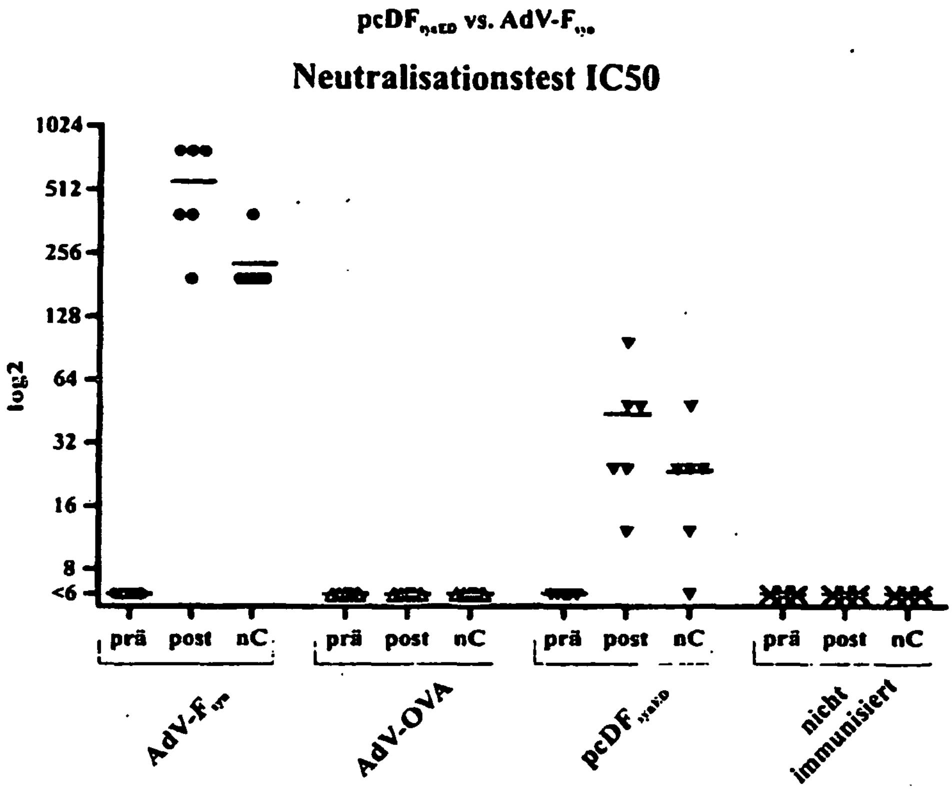

Neutralisationstest IC50
1024
512
256
128
64
32
16
8
<6
log2
prä
post
nC
AdV-OVA
nicht immunisiert


Figur 4

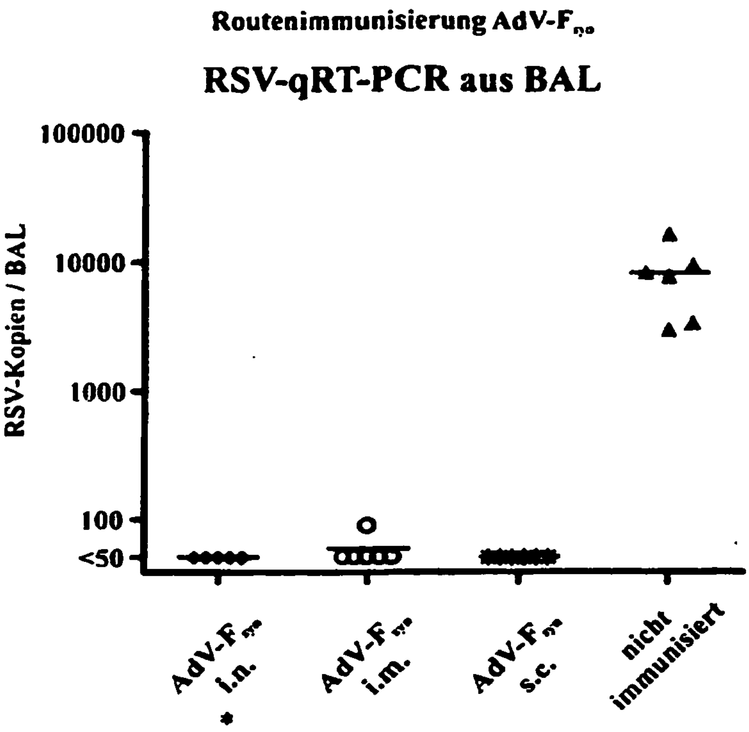
Routenimmunisierung AdV-F$_{co}$
RSV-qRT-PCR aus BAL
RSV-Kopien / BAL
100000
10000
1000
100
<50
AdV-F$_{co}$ i.n.
AdV-F$_{co}$ i.m.
AdV-F$_{co}$ s.c.
nicht immunisiert

Figur 5

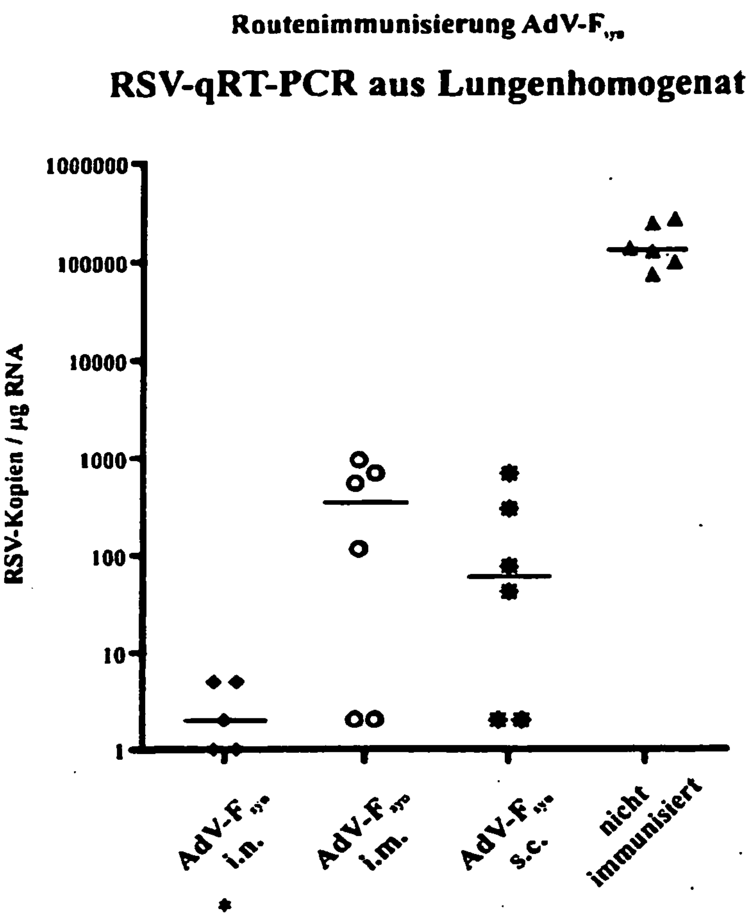
Routenimmunisierung AdV-F$_{syn}$
RSV-qRT-PCR aus Lungenhomogenat
RSV-Kopien / µg RNA
1000000
100000
10000
1000
100
10
1
AdV-F$_{syn}$ i.n.
AdV-F$_{syn}$ i.m.
AdV-F$_{syn}$ s.c.
nicht immunisiert

Figur 6

Routenimmunisierung AdV-F$_{vs}$
**IgG1-ELISA**

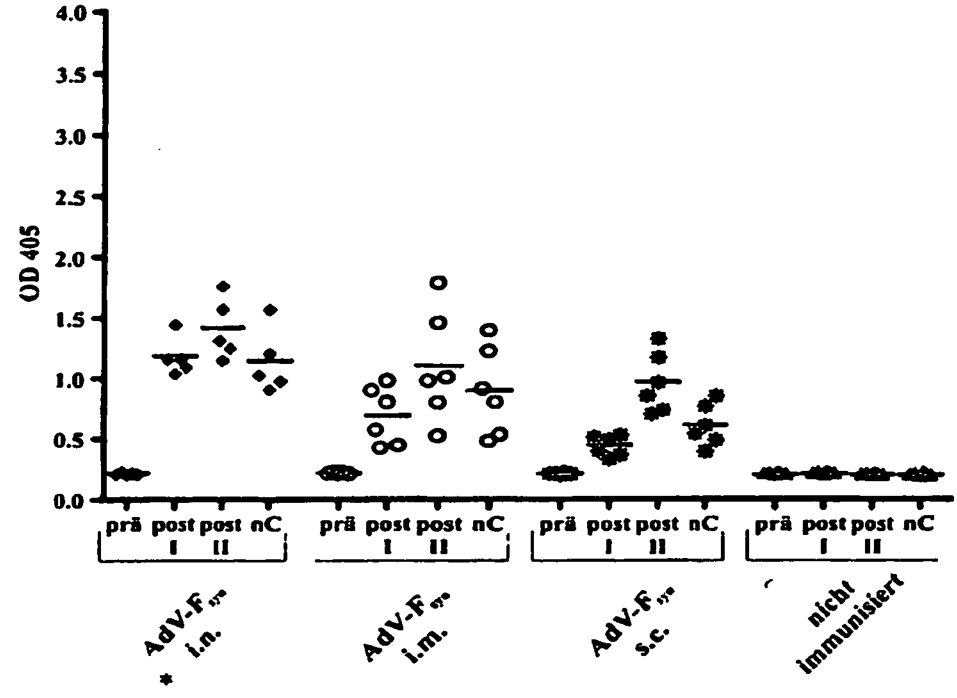


Routenimmunisierung AdV-F$_{vs}$
**IgG2a-ELISA**

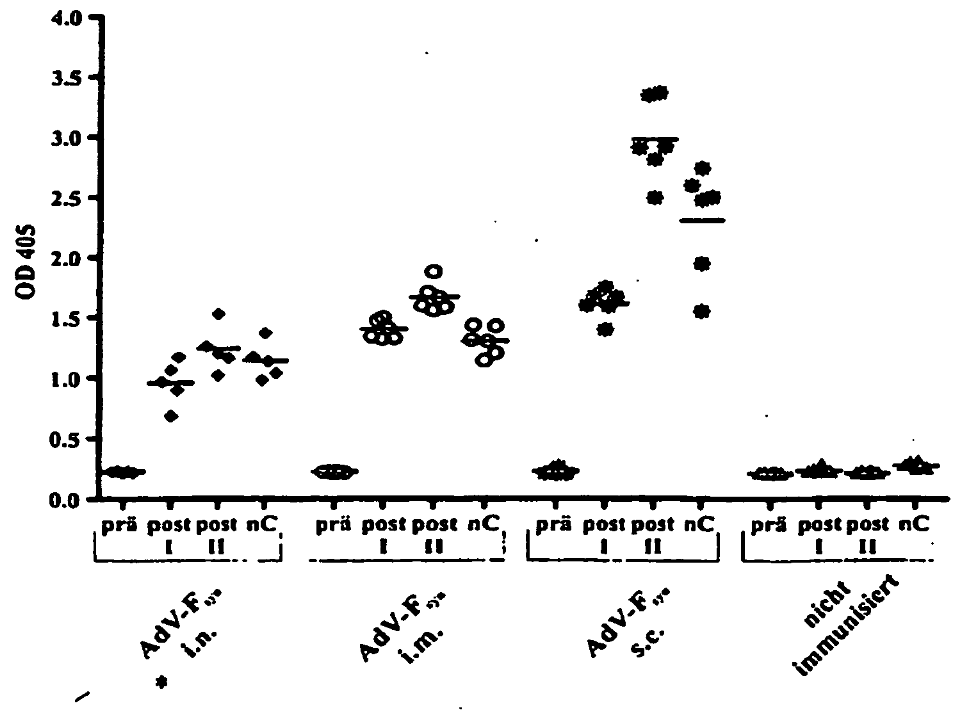


Figur 7

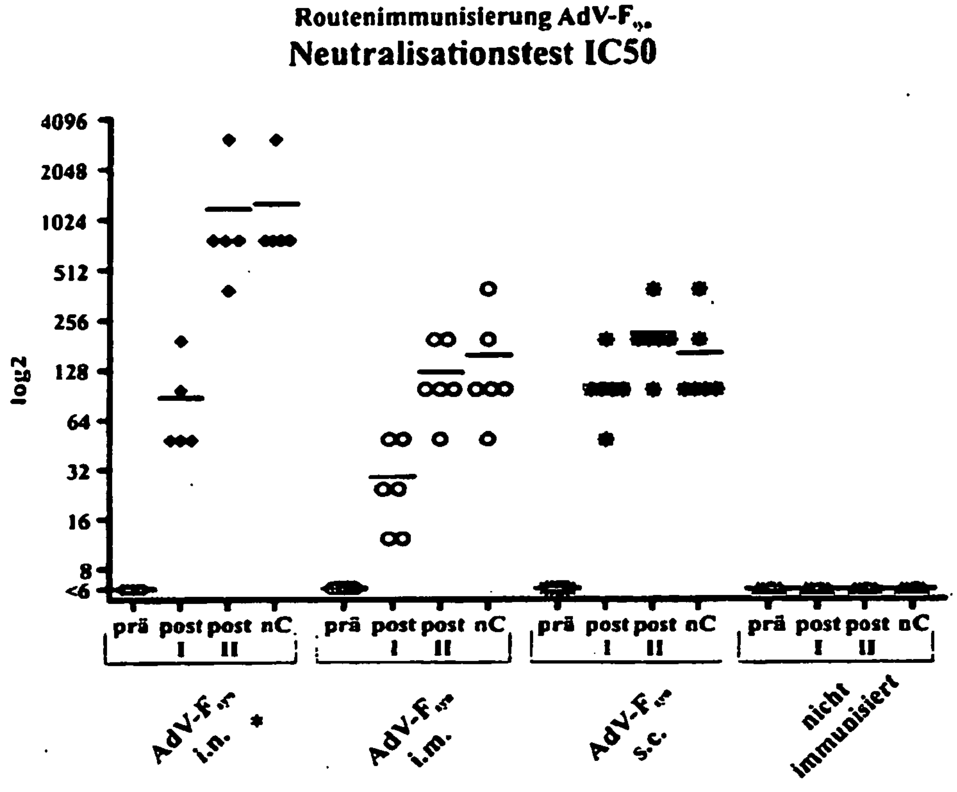
Routenimmunisierung AdV-F
Neutralisationstest IC50
4096
2048
1024
512
256
128
64
32
16
8
<6
log2
prä post I post II nC
prä post I post II nC
prä post I post II nC
prä post I post II nC
AdV-F i.n. *
AdV-F i.m.
AdV-F s.c.
nicht immunisiert

Figur 8

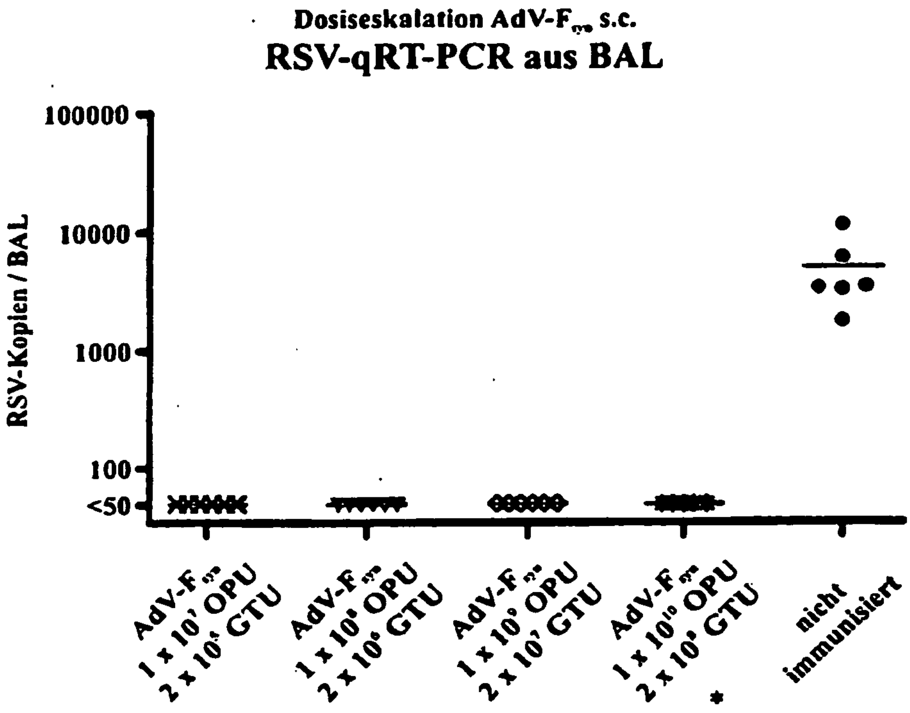
Dosiseskalation AdV-F s.c.
RSV-qRT-PCR aus BAL
RSV-Kopien / BAL
100000
10000
1000
100
<50
AdV-F 1 x 10⁷ OPU 2 x 10⁵ GTU
AdV-F 1 x 10⁸ OPU 2 x 10⁶ GTU
AdV-F 1 x 10⁹ OPU 2 x 10⁷ GTU
AdV-F 1 x 10¹⁰ OPU 2 x 10⁸ GTU
*
nicht immunisiert

Figur 9

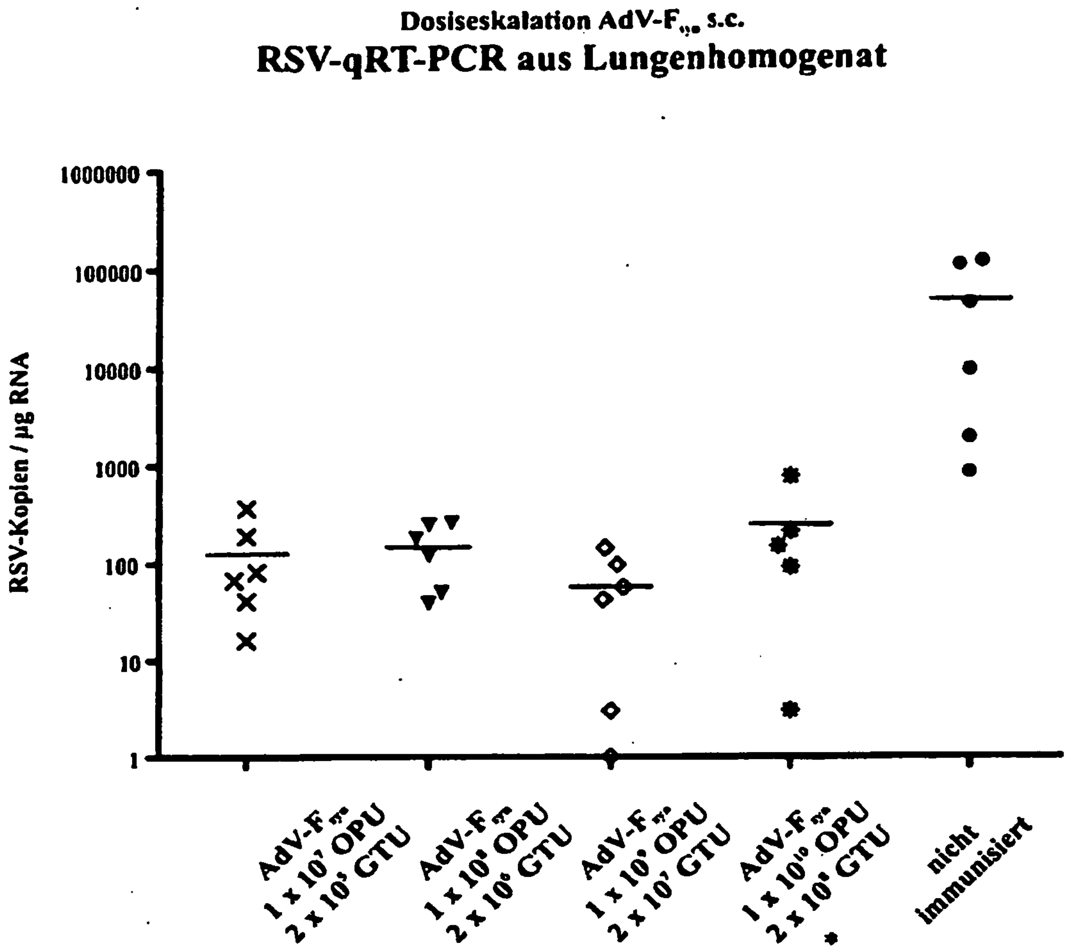
Dosiseskalation AdV-$F_{syn}$ s.c.
RSV-qRT-PCR aus Lungenhomogenat
RSV-Kopien / µg RNA
1000000
100000
10000
1000
100
10
1
AdV-$F_{syn}$ 1 x $10^7$ OPU 2 x $10^5$ GTU
AdV-$F_{syn}$ 1 x $10^8$ OPU 2 x $10^6$ GTU
AdV-$F_{syn}$ 1 x $10^9$ OPU 2 x $10^7$ GTU
AdV-$F_{syn}$ 1 x $10^{10}$ OPU 2 x $10^8$ GTU *
nicht immunisiert

Figur 10

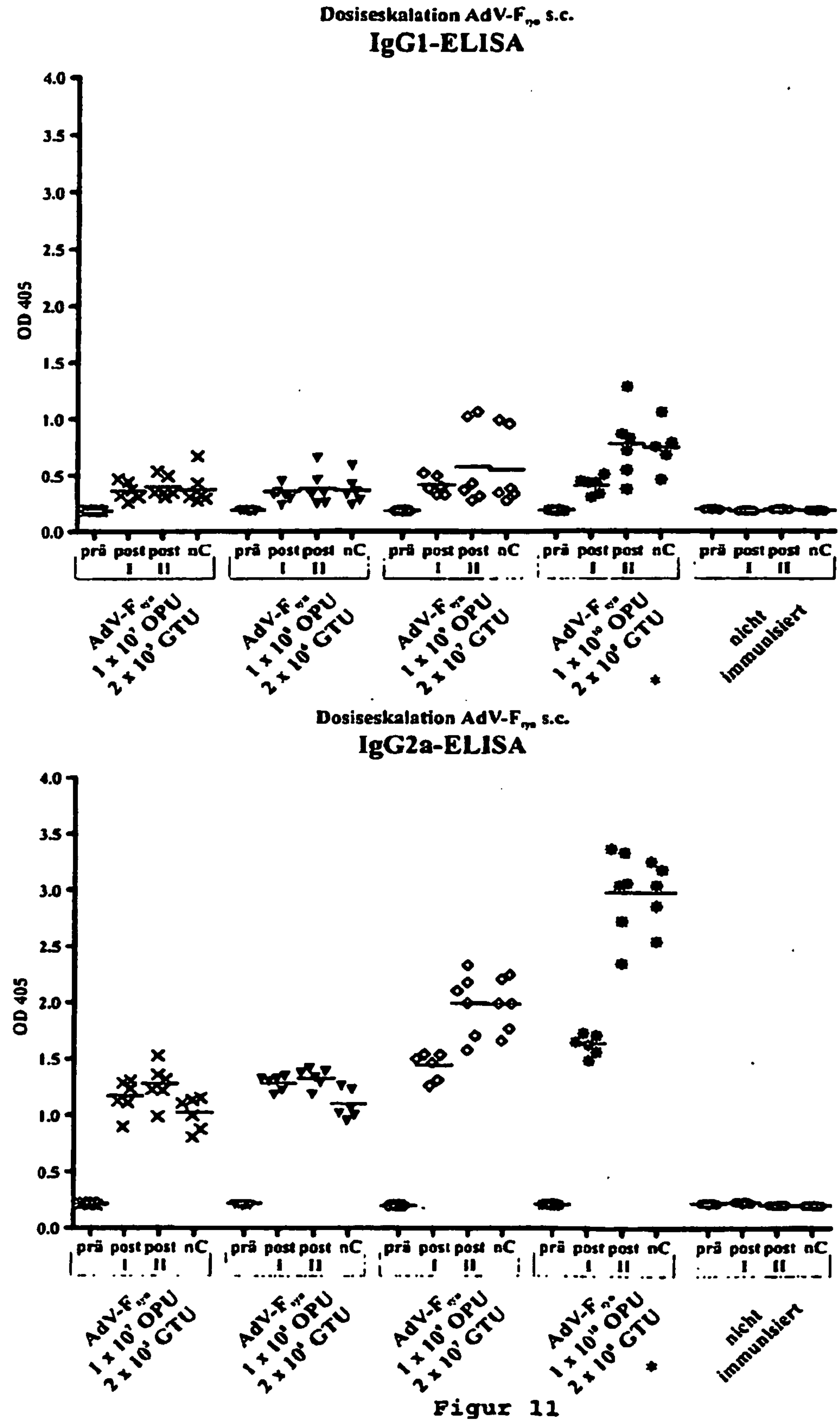
Dosiseskalation AdV-F s.c.
IgG1-ELISA
OD 405
4.0
3.5
3.0
2.5
2.0
1.5
1.0
0.5
0.0
prä post post nC
I II
nicht immunisiert
Dosiseskalation AdV-F s.c.
IgG2a-ELISA

Figur 11

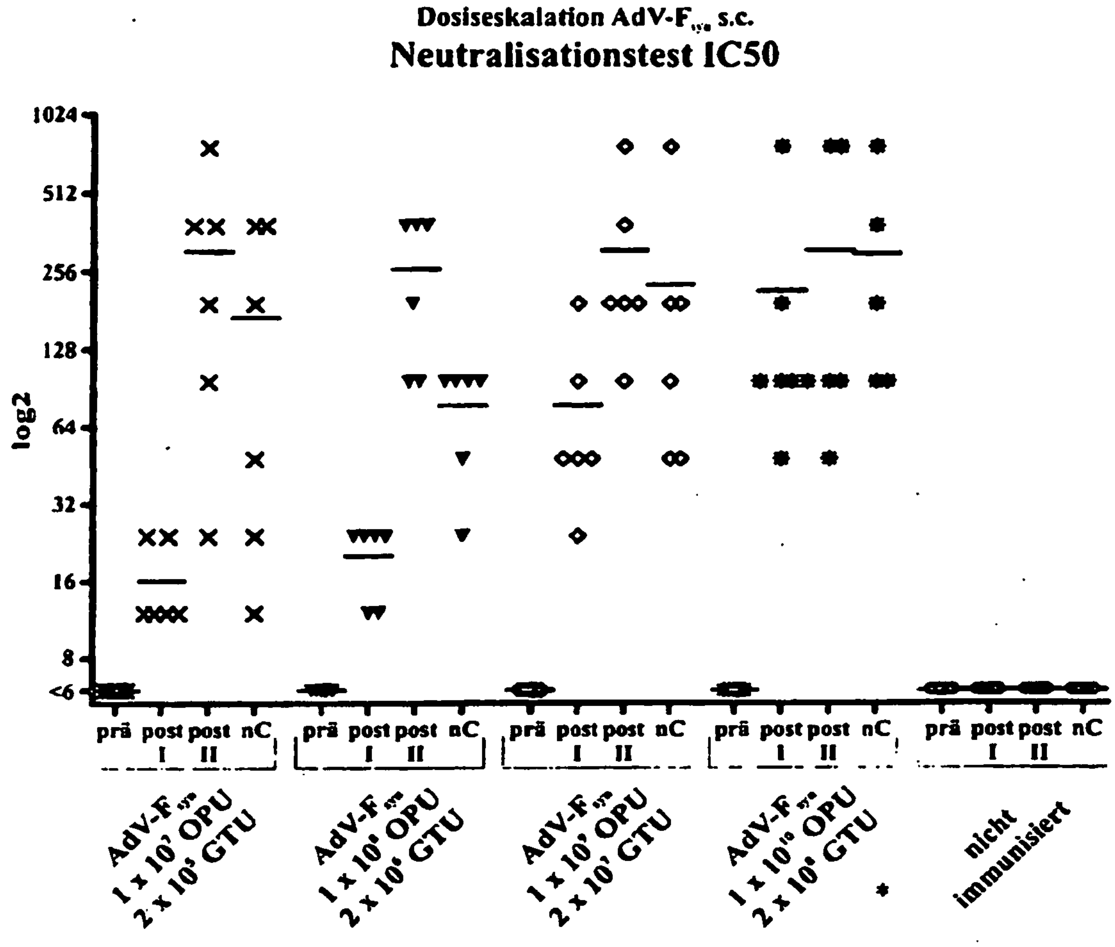
Dosiseskalation AdV-F s.c.
Neutralisationstest IC50
log2
1024
512
256
128
64
32
16
8
<6
prä post post nC
I II
AdV-F
1 x 10⁷ OPU
2 x 10⁵ GTU
AdV-F
1 x 10⁸ OPU
2 x 10⁶ GTU
AdV-F
1 x 10⁹ OPU
2 x 10⁷ GTU
AdV-F
1 x 10¹⁰ OPU
2 x 10⁸ GTU
nicht immunisiert

Figur 12

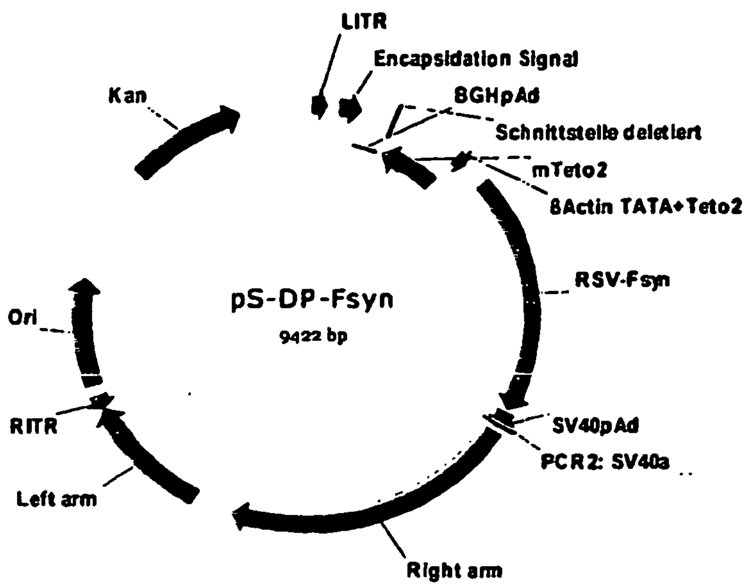
LITR
Encapsidation Signal
Kan
BGHpAd
Schnittstelle deletiert
mTeto2
ßActin TATA+Teto2
RSV-Fsyn
pS-DP-Fsyn
9422 bp
Ori
RITR
SV40pAd
PCR2: SV40a
Left arm
Right arm

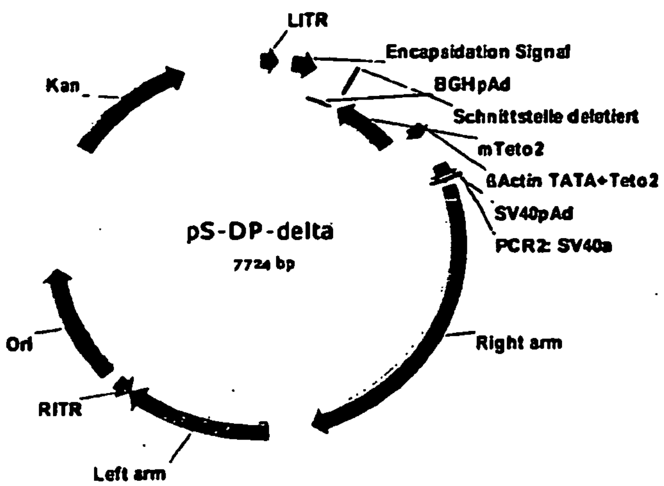
LITR
Encapsidation Signal
Kan
BGHpAd
Schnittstelle deletiert
mTeto2
ßActin TATA+Teto2
SV40pAd
PCR2: SV40a
pS-DP-delta
7724 bp
Right arm
Ori
RITR
Left arm

Figur 13

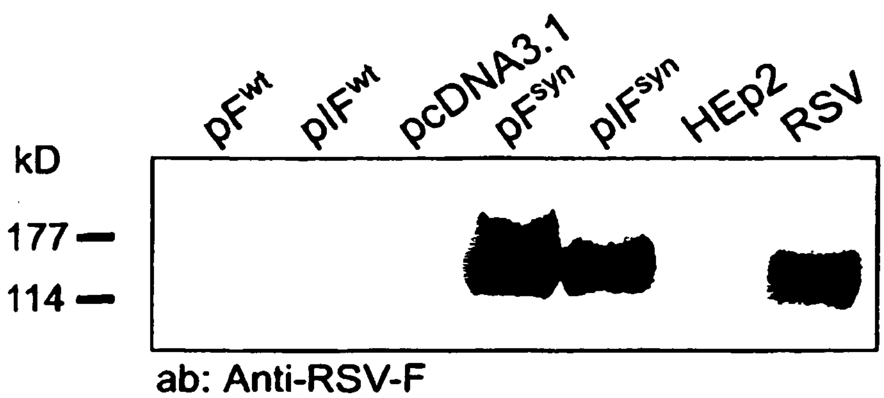
pF^wt
pIF^wt
pcDNA3.1
pF^syn
pIF^syn
HEp2
RSV
kD
177
114
ab: Anti-RSV-F

Figur 14

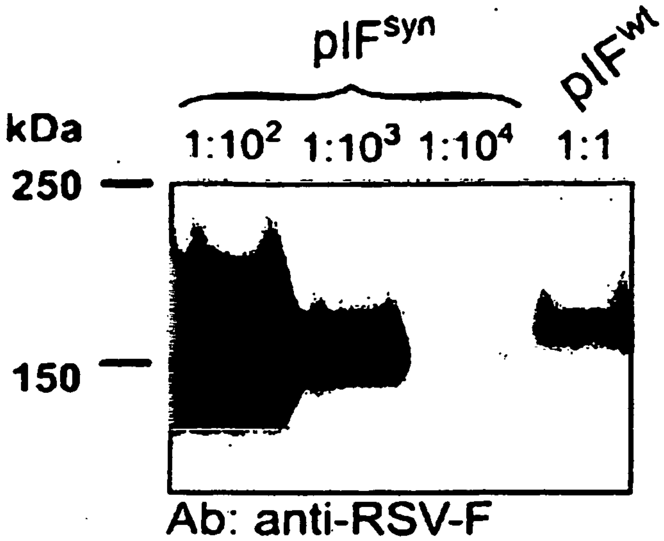


Figur 15

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0018929 A
- US 5922576 A


### In der Beschreibung aufgeführte Nicht-Patentliteratur


- **Park.** *Mol. & Cells,* 2001, vol. 12 (1), 50-56
- **Li.** *J. Exp. Med.,* 1998, vol. 188 (4), 681-688
- **Tree.** *Vaccine,* 2004, vol. 22 (19), 2438-2443
- Remington's Pharmaceutical Sciences. Williams & Wilkins PA, 2000
- **DuBridge et al.** Analysis of mutation in human cells by using an Epstein-Barr virus shuttle System. *Mol. Cell Biol.,* 1987, vol. 7, 379-387